# EUROPEAN PATENT APPLICATION

(11) **EP 4 696 296 A2**
(43) Date of publication of application: **18.02.2026**
(21) Application number: 25226409.8
(22) Date of filing: 29.05.2019
(51) Int. Cl.: A61J 1/20

(54) **PARENTERAL NUTRITION FORMULATION**

(30) Priority: 01.06.2018 US 201862679352 P
(62) Divisional of application: 19731456.0
(71) Applicant: BAXTER INTERNATIONAL INC., Deerfield, IL 60015-4634 (US); BAXTER HEALTHCARE SA, 8152 Glattpark (Opfikon) (CH)
(72) Inventor: Trouilly, Jean-Luc, 1420 Braine L'Alleud (BE); Hecq, Julien, 1180 Uccle (BE); Fahier, Julie, 1420 Braine L'Alleud (BE); Hise Brown, Mary, IL 60061, Vernon Hills (US); Jeannin, Laurent, 1180 Uccle (BE)
(74) Representative: SONN Patentanwälte GmbH & Co KG

(57) **Abstract**

Parenteral nutrition formulations for pediatric patients are disclosed. The nutritional formulations include a lipid formulation that comprises an aqueous phase and an oil phase, wherein the lipid formulation is in the form of an oil-in-water emulsion. Multi-chamber containers for parenteral nutrition administration comprising a carbohydrate formulation present in a first chamber, an amino acid formulation present in a second chamber, and the lipid formulation present in a third chamber also are disclosed.

## Description

### BACKGROUND

### FIELD

The disclosure is directed to parenteral nutrition formulations, including ready-to-use ternary parenteral nutrition formulations. More particularly, the present disclosure is directed to lipid formulations and to multi-chamber containers containing lipid formulations, carbohydrate formulations, and amino acid formulations, particularly for use with pediatric patients.

### DESCRIPTION OF THE RELATED ART

Medical solutions for pediatric nutrition need to be formulated with the needs of preterm babies, neonates, infants, children, and adolescents in mind. Preterm babies lack substantial energy and fat reserves, for example, making it important to provide proper lipid intake via parenteral nutrition. Lipids, particularly long-chain polyunsaturated fatty acids, have been found to be involved in growth, visual development, neural development, and long-term health, among other functions.

Docosahexaenoic acid (DHA) (C22:6 n-3) is a long-chain polyunsaturated fatty acid synthetized from α-linolenic acid (ALA). DHA is a major structural lipid for cellular membranes in the brain and the retina of the eyes, and is a key component of the heart. While the use of parenteral nutrition is associated with liver disease (e.g., liver failure, hepatic steatosis, cirrhosis), DHA was found to have a beneficial impact on liver steatosis.

Arachidonic acid (ARA) (C20:4 n-6) is a long-chain polyunsaturated fatty acid formed by biosynthesis from linoleic acid (LA). ARA is the most abundant fatty acid in the brain where it plays an important role in cell division, signaling and many basic cellular functions.

An improved parenteral nutrition product for pediatric patients, including preterm babies, neonates, infants, children, and adolescents, who require parenteral nutrition when oral and enteral nutrition is not possible, insufficient or contraindicated, is needed accordingly.

### SUMMARY

In light of the disclosure herein, and without limiting the scope of the invention in any way, in a first aspect of the present disclosure, which may be combined with any other aspect listed herein unless specified otherwise, a multi-chamber container for parenteral administration includes a carbohydrate formulation present in a first chamber, an amino acid formulation present in a second chamber, and a lipid formulation present in a third chamber. The lipid formulation has an aqueous phase and an oil phase, includes about 5% to about 35% by weight of the oil phase based on the total weight of the lipid formulation, and is present in the form of an oil-in-water emulsion. Furthermore, the oil phase comprises docosahexaenoic acid (DHA) obtained from a single cell source, the single cell source being an extract of microalgae. In the oil phase, DHA is present in a concentration of from 0.1 g to 5.0 g per 100 g of oil phase. The lipid formulation includes about 70 mg phytosterols or less per 100 g of oil phase, and is essentially free of water soluble forms of choline and/or arachidonic acid (ARA).

In a second aspect of the present disclosure, which may be combined with any other aspect listed herein unless specified otherwise, DHA is present in a concentration of from 0.25 g to 3.0 g per 100 g of oil phase.

In a third aspect of the present disclosure, which may be combined with any other aspect listed herein unless specified otherwise, DHA is present in a concentration of from 1.5 g to 2.5 g per 100 g of oil phase.

In a fourth aspect of the present disclosure, which may be combined with any other aspect listed herein unless specified otherwise, DHA is present in triglyceride form or ethyl ester form, preferably triglyceride form.

In a fifth aspect of the present disclosure, which may be combined with any other aspect listed herein unless specified otherwise, the lipid formulation includes eicosapentaenoic acid (EPA) and the ratio of DHA to EPA in the lipid formulation is 10:1 to 1000:1 (w/w).

In a sixth aspect of the present disclosure, which may be combined with any other aspect listed herein unless specified otherwise, the lipid formulation includes EPA and the ratio of DHA to EPA in the lipid formulation is 10:1 to 200:1.

In a seventh aspect of the present disclosure, which may be combined with any other aspect listed herein unless specified otherwise, the lipid formulation includes EPA and the ratio of DHA to EPA in the lipid formulation is 20:1 to 150:1.

In an eighth aspect of the present disclosure, which may be combined with any other aspect listed herein unless specified otherwise, DHA and/or EPA are present in triglyceride form or ethyl ester form, preferably triglyceride form.

In a ninth aspect of the present disclosure, which may be combined with any other aspect listed herein unless specified otherwise, the lipid formulation is essentially free of EPA.

In an tenth aspect of the present disclosure, which may be combined with any other aspect listed herein unless specified otherwise, the microalgae is *Crypthecodinium cohnii* or *Schizochytrium sp,* preferably *Schizochytrium sp.*

In an eleventh aspect of the present disclosure, which may be combined with any other aspect listed herein unless specified otherwise, the lipid formulation includes about 15 mg to about 35 mg phytosterols per 100 g of oil phase, and in one preferred embodiment, about 25 mg phytosterols or less per 100 g of oil phase.

In a twelfth aspect of the present disclosure, which may be combined with any other aspect listed herein unless specified otherwise, the lipid formulation includes one or more pharmaceutically acceptable surfactants selected from the group consisting of phospholipids, oleate salts, and combinations thereof.

In a thirteenth aspect of the present disclosure, which may be combined with any other aspect listed herein unless specified otherwise, the phospholipid is selected from the group consisting of egg phosphatide and soy lecithin.

In a fourteenth aspect of the present disclosure, which may be combined with any other aspect listed herein unless specified otherwise, the lipid formulation includes one or more pharmaceutically acceptable isotonic agents.

In a fifteenth aspect of the present disclosure, which may be combined with any other aspect listed herein unless specified otherwise, the pharmaceutically acceptable isotonic agent is glycerol.

In a sixteenth aspect of the present disclosure, which may be combined with any other aspect listed herein unless specified otherwise, the lipid formulation includes one or more pharmaceutically acceptable antioxidants selected from the group consisting of tocopherols, ascorbyl palmitate, and combinations thereof.

In a seventeenth aspect of the present disclosure, which may be combined with any other aspect listed herein unless specified otherwise, the lipid formulation has a pH in the range of about 6 to about 9, and in one preferred embodiment, about 7 to about 8.

In a eighteenth aspect of the present disclosure, which may be combined with any other aspect listed herein unless specified otherwise, the carbohydrate formulation and/or the amino acid formulation includes at least one water-soluble form of choline selected from the group consisting of choline chloride, choline bitartrate, choline citrate, choline gluconate, choline malate, choline cytidine diphosphate choline (CDP) salt and glycerophosphocholine, preferably glycerophosphocholine.

In a nineteenth aspect of the present disclosure, which may be combined with any other aspect listed herein unless specified otherwise, the at least one choline is present in a concentration of from 20 mg to 2000 mg choline equivalent per liter of a reconstituted multi-chamber container, such as 30 mg to 1000 mg choline equivalent per liter of a reconstituted multi-chamber container.

In a twentieth aspect of the present disclosure, which may be combined with any other aspect listed herein unless specified otherwise, a method of treating patients who require parenteral nutrition when oral and enteral nutrition is not possible, insufficient or contraindicated includes parenterally administering the contents of a multi-chamber container that includes a lipid formulation. The lipid formulation has an aqueous phase and an oil phase, includes about 5% to about 35% by weight of the oil phase based on the total weight of the lipid formulation, and is present in the form of an oil-in-water emulsion. Furthermore, the oil phase comprises docosahexaenoic acid (DHA) obtained from a single cell source, the single cell source being an extract of microalgae. In the oil phase, DHA is present in a concentration of from 0.1 g to 5.0 g per 100 g of oil phase. The lipid formulation includes about 70 mg phytosterols or less per 100 g of oil phase, and is essentially free of water soluble forms of choline and/or arachidonic acid (ARA).

In a twenty-first aspect of the present disclosure, which may be combined with any other aspect listed herein unless specified otherwise, the patients are pediatric patients.

In a twenty-second aspect of the present disclosure, which may be combined with any other aspect listed herein unless specified otherwise, a lipid formulation for parenteral administration includes an aqueous phase and an oil phase. The lipid formulation includes about 5% to about 35% by weight of the oil phase based on the total weight of the lipid formulation, and is present in the form of an oil-in-water emulsion. Furthermore, the aqueous phase includes at least one pharmaceutically acceptable water soluble form of choline. The oil phase includes docosahexaenoic acid (DHA). The lipid formulation includes about 70 mg phytosterols or less per 100 g of oil phase, and is essentially free of arachidonic acid (ARA).

In a twenty-third aspect of the present disclosure, which may be combined with any other aspect listed herein unless specified otherwise, the DHA is obtained from a single cell source, the single cell source being an extract of microalgae.

In a twenty-fourth aspect of the present disclosure, which may be combined with any other aspect listed herein unless specified otherwise, the microalgae is *Crypthecodinium cohnii* or *Schizochytrium sp,* preferably *Schizochytrium sp..*

In a twenty-fifth aspect of the present disclosure, which may be combined with any other aspect listed herein unless specified otherwise, DHA is present in a concentration of from 0.25 g to 3.0 g per 100 g of oil phase.

In a twenty-sixth aspect of the present disclosure, which may be combined with any other aspect listed herein unless specified otherwise, DHA is present in a concentration of from 1.5 g to 2.5 g per 100 g of oil phase.

In a twenty-seventh aspect of the present disclosure, which may be combined with any other aspect listed herein unless specified otherwise, DHA is present in triglyceride form or ethyl ester form, preferably triglyceride form.

In a twenty-eighth aspect of the present disclosure, which may be combined with any other aspect listed herein unless specified otherwise, the lipid formulation includes eicosapentaenoic acid (EPA) and the ratio of DHA to EPA in the lipid formulation is 10:1 to 1000:1 (w/w).

In a twenty-ninth aspect of the present disclosure, which may be combined with any other aspect listed herein unless specified otherwise, the lipid formulation includes EPA and the ratio of DHA to EPA in the lipid formulation is 10:1 to 200:1 (w/w).

In a thirtieth aspect of the present disclosure, which may be combined with any other aspect listed herein unless specified otherwise, the lipid formulation includes EPA and the ratio of DHA to EPA in the lipid formulation is 20:1 to 150:1 (w/w).

In a thirty-first aspect of the present disclosure, which may be combined with any other aspect listed herein unless specified otherwise, DHA and/or EPA are present in triglyceride form or ethyl ester form, preferably triglyceride form.

In a thirty-second aspect of the present disclosure, which may be combined with any other aspect listed herein unless specified otherwise, the lipid formulation is essentially free of EPA.

In a thirty-third aspect of the present disclosure, which may be combined with any other aspect listed herein unless specified otherwise, the lipid formulation includes 15 mg to 35 mg phytosterols per 100 g of oil phase, and in one preferred embodiment, about 25 mg phytosterols or less per 100 g oil phase.

In a thirty-fourth aspect of the present disclosure, which may be combined with any other aspect listed herein unless specified otherwise, the lipid formulation includes one or more pharmaceutically acceptable surfactants selected from the group consisting of phospholipids, oleate salts, and combinations thereof.

In a thirty-fifth aspect of the present disclosure, which may be combined with any other aspect listed herein unless specified otherwise, the phospholipid is selected from the group consisting of egg phosphatide and soy lecithin.

In a thirty-sixth aspect of the present disclosure, which may be combined with any other aspect listed herein unless specified otherwise, the lipid formulation includes one or more pharmaceutically acceptable isotonic agents.

In a thirty-seventh aspect of the present disclosure, which may be combined with any other aspect listed herein unless specified otherwise, the pharmaceutically acceptable isotonic agent is glycerol.

In a thirty-eighth aspect of the present disclosure, which may be combined with any other aspect listed herein unless specified otherwise, the lipid formulation includes one or more pharmaceutically acceptable antioxidants selected from the group consisting of tocopherols, ascorbyl palmitate, and combinations thereof.

In a thirty-ninth aspect of the present disclosure, which may be combined with any other aspect listed herein unless specified otherwise, the lipid formulation has a pH in the range of about 6 to about 9, and in one preferred embodiment, about 7 to about 8.

In a fortieth aspect of the present disclosure, which may be combined with any other aspect listed herein unless specified otherwise, the at least one choline is selected from the group consisting of choline chloride, choline bitartrate, choline citrate, choline gluconate, choline malate, choline cytidine diphosphate choline (CDP) salt and glycerophosphocholine, preferably glycerophosphocholine.

In a forty-first aspect of the present disclosure, which may be combined with any other aspect listed herein unless specified otherwise, the at least one choline is present in a concentration of from 0.1 g to 12 g of choline equivalent per liter of lipid emulsion, for example, from 0.2 g to 10 g of choline equivalent per liter of lipid emulsion, from 3 g to 6 g of choline equivalent per liter of lipid emulsion, from 0.5 to 4 g of choline equivalent per liter of lipid emulsion, from 0.1 g to 0.5 g of choline equivalent per liter of lipid emulsion, from 0.5 g to 1 g of choline equivalent per liter of lipid emulsion, from 1 g to 2 g of choline equivalent per liter of lipid emulsion, from 2 g to 3 g of choline equivalent per liter of lipid emulsion, from 3 g to 4 g of choline equivalent per liter of lipid emulsion, from 4 g to 5 g of choline equivalent per liter of lipid emulsion, from 5 g to 6 g of choline equivalent per liter of lipid emulsion, from 6 g to 7 g of choline equivalent per liter of lipid emulsion, from 7 g to 8 g of choline equivalent per liter of lipid emulsion, from 8 g to 9 g of choline equivalent per liter of lipid emulsion, from 9 g to 10 g of choline equivalent per liter of lipid emulsion, from 10 g to 11 g of choline equivalent per liter of lipid emulsion, and/or from 11 g to 12 g of choline equivalent per liter of lipid emulsion.

In a forty-second aspect of the present disclosure, which may be combined with any other aspect listed herein unless specified otherwise, the at least one choline is present in a concentration of from 0.2 g to 10 g of choline equivalent per liter of lipid emulsion.

In a forty-third aspect of the present disclosure, which may be combined with any other aspect listed herein unless specified otherwise, the at least one choline is present in a concentration of from 3 g to 6 g of choline equivalent per liter of lipid emulsion.

In a forty-fourth aspect of the present disclosure, which may be combined with any other aspect listed herein unless specified otherwise, a method of treating patients who require parenteral nutrition when oral and enteral nutrition is not possible, insufficient or contraindicated includes parenterally administering a lipid formulation. The lipid formulation includes an aqueous phase and an oil phase, about 5% to about 35% by weight of an oil phase based on the total weight of the lipid formulation, and is present in the form of an oil-in-water emulsion. Furthermore, the aqueous phase includes at least one pharmaceutically acceptable water soluble form of choline. The oil phase includes docosahexaenoic acid (DHA). The lipid formulation includes about 70 mg phytosterols or less per 100 g of oil phase, and is essentially free of arachidonic acid (ARA).

In a forty-fifth aspect of the present disclosure, which may be combined with any other aspect listed herein unless specified otherwise, the patients are pediatric patients.

In a forty-sixth aspect of the present disclosure, which may be combined with any other aspect listed herein unless specified otherwise, a multi-chamber container for parenteral administration includes a carbohydrate formulation present in a first chamber, an amino acid formulation present in a second chamber, and a lipid formulation present in a third chamber. The lipid formulation includes an aqueous phase and an oil phase, about 5% to about 35% by weight of an oil phase based on the total weight of the lipid formulation, and is present in the form of an oil-in-water emulsion. Furthermore, the aqueous phase includes at least one pharmaceutically acceptable water soluble form of choline. The oil phase includes docosahexaenoic acid (DHA). The lipid formulation includes about 70 mg phytosterols or less per 100 g of oil phase, and is essentially free of arachidonic acid (ARA).

In a forty-seventh aspect of the present disclosure, which may be combined with any other aspect listed herein unless specified otherwise, a lipid formulation for parenteral administration includes an aqueous phase and an oil phase. The lipid formulation includes about 5% to about 35% by weight of the oil phase based on the total weight of the lipid formulation, and is present in the form of an oil-in-water emulsion. Furthermore, the aqueous phase includes at least one pharmaceutically acceptable water soluble form of choline. The oil phase includes docosahexaenoic acid (DHA) and arachidonic acid (ARA), and the ratio of DHA to ARA in the lipid formulation is from 10:1 to 1:5 (w/w). The lipid formulation includes about 70 mg phytosterols or less per 100 g of oil phase.

In a forty-eighth aspect of the present disclosure, which may be combined with any other aspect listed herein unless specified otherwise, the ratio of DHA to ARA in the lipid formulation is 1:1 to 1:3 (w/w).

In a forty-ninth aspect of the present disclosure, which may be combined with any other aspect listed herein unless specified otherwise, DHA is obtained from a single cell source, wherein the single source is an extract of microalgae.

In a fiftieth aspect of the present disclosure, which may be combined with any other aspect listed herein unless specified otherwise, the microalgae is *Crypthecodinium cohnii* or *Schizochytrium sp,* preferably *Schizochytrium sp.*

In a fifty-first aspect of the present disclosure, which may be combined with any other aspect listed herein unless specified otherwise, ARA is obtained from a single cell source, wherein the cell source is fungi.

In a fifty-second aspect of the present disclosure, which may be combined with any other aspect listed herein unless specified otherwise, the fungus is *Mortierella alpina.*

In a fifty-third aspect of the present disclosure, which may be combined with any other aspect listed herein unless specified otherwise, DHA and/or ARA are present in triglyceride form or ethyl ester form, preferably triglyceride form.

In a fifty-fourth aspect of the present disclosure, which may be combined with any other aspect listed herein unless specified otherwise, ARA is present in a concentration range of from 0.1 g to 15 g per 100 g of oil phase.

In a fifty-fifth aspect of the present disclosure, which may be combined with any other aspect listed herein unless specified otherwise, ARA is present in a concentration range of from 1.5 g to 7.5 g per 100 g of oil phase.

In a fifty-sixth aspect of the present disclosure, which may be combined with any other aspect listed herein unless specified otherwise, DHA is present in a concentration of from 0.25 g to 3.0 g per 100 g of oil phase.

In a fifty-seventh aspect of the present disclosure, which may be combined with any other aspect listed herein unless specified otherwise, DHA is present in a concentration of from 1.5 g to 2.5 g per 100 g of oil phase.

In a fifty-eighth aspect of the present disclosure, which may be combined with any other aspect listed herein unless specified otherwise, the lipid formulation comprises EPA and wherein the ratio of DHA to EPA in the lipid formulation is 10:1 to 1000:1 (w/w).

In a fifty-ninth aspect of the present disclosure, which may be combined with any other aspect listed herein unless specified otherwise, the lipid formulation comprises EPA and the ratio of DHA to EPA in the lipid formulation is 10:1 to 200:1 (w/w).

In a sixtieth aspect of the present disclosure, which may be combined with any other aspect listed herein unless specified otherwise, the lipid formulation comprises EPA and the ratio of DHA to EPA in the lipid formulation is 20:1 to 150:1 (w/w).

In a sixty-first aspect of the present disclosure, which may be combined with any other aspect listed herein unless specified otherwise, DHA and/or EPA are present in triglyceride form or ethyl ester form, preferably triglyceride form.

In a sixty-second aspect of the present disclosure, which may be combined with any other aspect listed herein unless specified otherwise, the lipid formulation is essentially free of EPA.

In a sixty-third aspect of the present disclosure, which may be combined with any other aspect listed herein unless specified otherwise, the lipid formulation includes 15 mg to 35 mg phytosterols per 100 g of oil phase, and in one preferred embodiment, about 25 mg phytosterols or less per 100 g oil phase.

In a sixty-fourth aspect of the present disclosure, which may be combined with any other aspect listed herein unless specified otherwise, the lipid formulation comprises one or more pharmaceutically acceptable surfactants selected from the group consisting of phospholipids, oleate salts, and combinations thereof.

In a sixty-fifth aspect of the present disclosure, which may be combined with any other aspect listed herein unless specified otherwise, the phospholipid is selected from the group consisting of egg phosphatide and soy lecithin.

In a sixty-sixth aspect of the present disclosure, which may be combined with any other aspect listed herein unless specified otherwise, the lipid formulation comprises one or more pharmaceutically acceptable isotonic agents.

In a sixty-seventh aspect of the present disclosure, which may be combined with any other aspect listed herein unless specified otherwise, the pharmaceutically acceptable isotonic agent is glycerol.

In a sixty-eighth aspect of the present disclosure, which may be combined with any other aspect listed herein unless specified otherwise, the lipid formulation includes one or more pharmaceutically acceptable antioxidants selected from the group consisting of tocopherols, ascorbyl palmitate, and combinations thereof.

In a sixty-ninth aspect of the present disclosure, which may be combined with any other aspect listed herein unless specified otherwise, the lipid formulation has a pH in the range of about 6 to about 9, and in one preferred embodiment, about 7 to about 8.

In a seventieth aspect of the present disclosure, which may be combined with any other aspect listed herein unless specified otherwise, the at least one water soluble form of choline is selected from the group consisting of choline chloride, choline bitartrate, choline citrate, choline gluconate, choline malate, choline cytidine diphosphate choline (CDP) salt and glycerophosphocholine.

In a seventy-first aspect of the present disclosure, which may be combined with any other aspect listed herein unless specified otherwise, the at least one choline is present in a concentration of from 0.1 g to 12 g of choline equivalent per liter of lipid emulsion, for example, from 0.2 g to 10 g of choline equivalent per liter of lipid emulsion, from 3 g to 6 g of choline equivalent per liter of lipid emulsion, from 0.5 to 4 g of choline equivalent per liter of lipid emulsion, from 0.1 g to 0.5 g of choline equivalent per liter of lipid emulsion, from 0.5 g to 1 g of choline equivalent per liter of lipid emulsion, from 1 g to 2 g of choline equivalent per liter of lipid emulsion, from 2 g to 3 g of choline equivalent per liter of lipid emulsion, from 3 g to 4 g of choline equivalent per liter of lipid emulsion, from 4 g to 5 g of choline equivalent per liter of lipid emulsion, from 5 g to 6 g of choline equivalent per liter of lipid emulsion, from 6 g to 7 g of choline equivalent per liter of lipid emulsion, from 7 g to 8 g of choline equivalent per liter of lipid emulsion, from 8 g to 9 g of choline equivalent per liter of lipid emulsion, from 9 g to 10 g of choline equivalent per liter of lipid emulsion, from 10 g to 11 g of choline equivalent per liter of lipid emulsion, and/or from 11 g to 12 g of choline equivalent per liter of lipid emulsion.

In a seventy-second aspect of the present disclosure, which may be combined with any other aspect listed herein unless specified otherwise, the at least one choline is present in a concentration of from 0.2 g to 10 g of choline equivalent per liter of lipid emulsion.

In a seventy-third aspect of the present disclosure, which may be combined with any other aspect listed herein unless specified otherwise, the at least one choline is present in a concentration of from 3 g to 6 g of choline equivalent per liter of lipid emulsion.

In a seventy-fourth aspect of the present disclosure, which may be combined with any other aspect listed herein unless specified otherwise, the choline is present in a concentration of from 0.5 to 4 g of choline equivalent per liter of lipid emulsion.

In a seventy-fifth aspect of the present disclosure, which may be combined with any other aspect listed herein unless specified otherwise, a method of treating pediatric patients who require parenteral nutrition when oral and enteral nutrition is not possible, insufficient or contraindicated includes parenterally administering a lipid formulation. The lipid formulation includes an aqueous phase and an oil phase, about 5% to about 35% by weight of the oil phase based on the total weight of the lipid formulation, and is present in the form of an oil-in-water emulsion. Furthermore, the aqueous phase includes at least one pharmaceutically acceptable water soluble form of choline. The oil phase includes docosahexaenoic acid (DHA) and arachidonic acid (ARA), and the ratio of DHA to ARA in the lipid formulation is from 10:1 to 1:5 (w/w). The lipid formulation includes about 70 mg phytosterols or less per 100 g of oil phase.

In a seventy-sixth aspect of the present disclosure, which may be combined with any other aspect listed herein unless specified otherwise, a multi-chamber container for parenteral administration includes a carbohydrate formulation present in a first chamber, an amino acid formulation present in a second chamber, and a lipid formulation present in a third chamber. The lipid formulation includes an aqueous phase and an oil phase, about 5% to about 35% by weight of the oil phase based on the total weight of the lipid formulation, and is present in the form of an oil-in-water emulsion. Furthermore, the aqueous phase includes at least one pharmaceutically acceptable water soluble form of choline. The oil phase includes docosahexaenoic acid (DHA) and arachidonic acid (ARA), and the ratio of DHA to ARA in the lipid formulation is from 10:1 to 1:5 (w/w). The lipid formulation includes about 70 mg phytosterols or less per 100 g of oil phase

In light of the disclosure and aspects set forth herein, it is accordingly an advantage of the present disclosure to provide a parenteral nutrition formulation that better meets the requirements of pediatric patients.

It is another advantage of the present disclosure to provide a beneficial impact on visual, somatic, and/or neural development.

It is a further advantage of the present disclosure to reduce parenteral nutrition-associated liver disease (PNALD), which is associated with liver failure, hepatic steatosis, and cirrhosis.

It is yet another advantage of the present disclosure to reduce non-alcoholic fatty liver disease (NAFLD).

It is yet a further advantage of the present disclosure to reduce chronic liver disease.

It is still a further advantage of the present disclosure to deplete phytosterols.

Additional features and advantages of the disclosed formulations are described in, and will be apparent from, the following Detailed Description and the Figures. The features and advantages described herein are not all-inclusive and, in particular, many additional features and advantages will be apparent to one of ordinary skill in the art in view of the figures and description. Also, any particular embodiment does not necessarily have to have all of the advantages listed herein. Moreover, it should be noted that the language used in the specification has been principally selected for readability and instructional purposes, and not to limit the scope of the inventive subject matter.

### BRIEF DESCRIPTION OF THE FIGURES

Understanding that figures depict only typical embodiments of the invention and are not to be considered to be limiting the scope of the present disclosure, the present disclosure is described and explained with additional specificity and detail through the use of the accompanying figures.

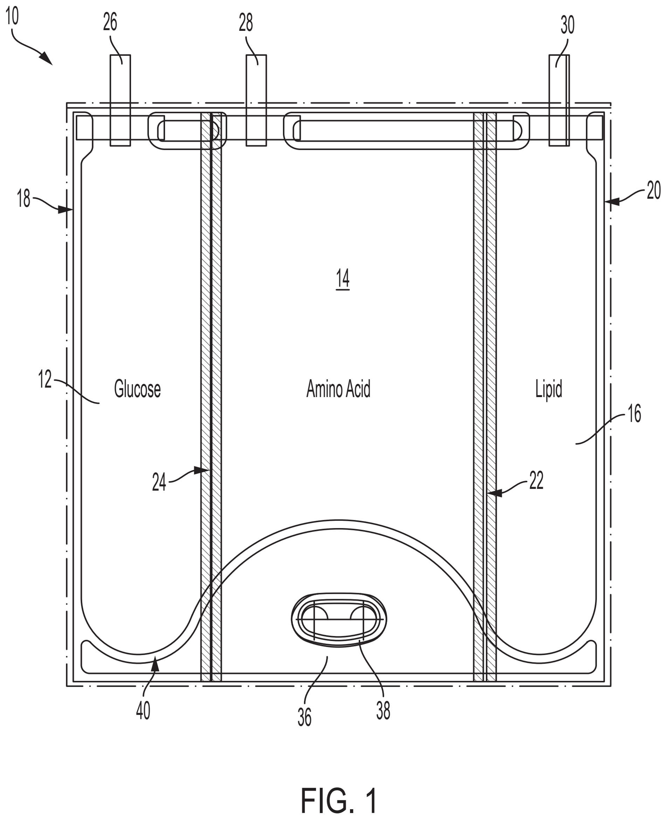

Figure 1 is a plan view of one embodiment of a 300 ml container of the present invention.

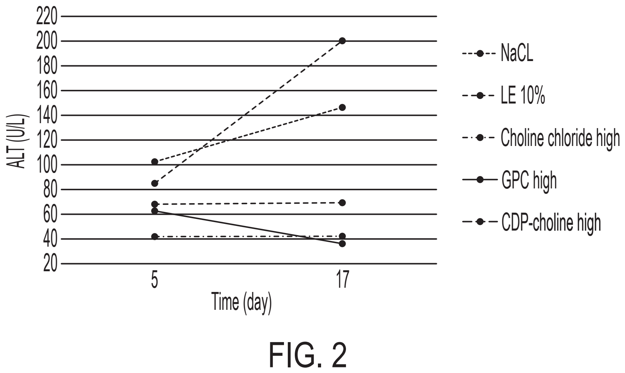

Figure 2 depicts the development of the alanine aminotransferase (ALT) function in the animal study described in Example 1. Control groups received saline solution (NaCl) or lipid emulsion LE 10% only, whereas further groups received the same LE 10% lipid emulsion, however with three different choline sources, choline chloride, CDP-choline and GPC contained in the lipid emulsion. The expression "high" denotes a concentration of the choline derivative of 31 mmol/L in the LE 10% lipid emulsion. The graph shows that the drop of ALT activity from day 5 to day 17, demonstrating an improvement of the liver condition, is most pronounced when GPC is present in the lipid emulsion.

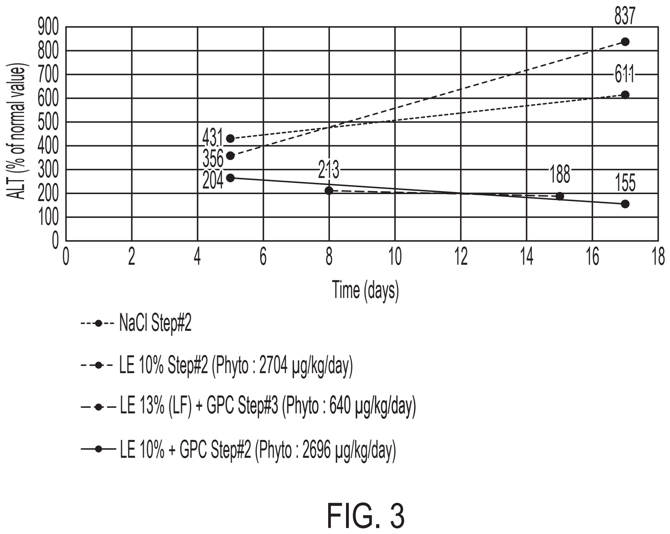

Figure 3 shows the development of the alanine aminotransferase activity in per cent of the normal value for plasma in an animal study as described in Example 1 (Step#3). The animals tested received either saline solution or LE 10% lipid emulsion ("LE 10%") only, wherein the lipid emulsion contained normal amounts of phytosterol ("Phyto") but no GPC. Another lipid emulsion, containing also LE 10% and comparable amounts of phytosterol, further contained GPC in a concentration of 40 mmol/L ("LE 10% + GPC Step#2"). A third composition was again consisting of a lipid emulsion (LE 13%) with GPC added at a concentration of 40 mmol/L and a reduced phytosterol content (phytosterol doses are given in Figure 3).

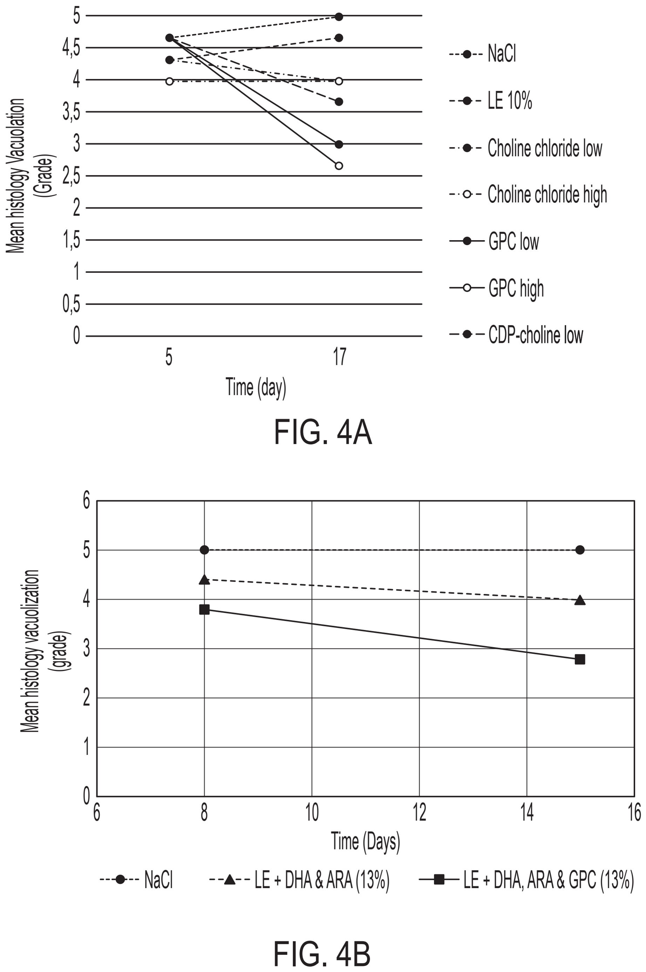

Figure 4A and Figure 4B represent the results of the animal study as described in Example 1 and the histopathological evaluation of the livers of animals that had been subjected to parenteral nutrition with saline solution, LE 10% lipid emulsion, and LE 10% lipid emulsions supplemented with choline chloride, CDP-choline and GPC, respectively (Figure 4A). Figure 4A shows that the administration of a lipid emulsion comprising GPC has the most significant impact on the development of vacuolation in comparison with all choline derivatives tested. Figure 4B provides for the results of administering again saline solution alone, a lipid emulsions (LE 13%) which has been supplemented with DHA and ARA (triangles) and a lipid emulsion (LE 13%) which has been supplemented with DHA, ARA and GPC (squares). Figure 4B shows that the presence of DHA and ARA already has a significant positive effect on liver vacuolation. The expression "low" refers to a concentration of the respective choline derivative of 15 mmol/L. The expression "high" refers to a choline derivative concentration of 31 mmol/L. Grades are defined as provided for in Example 3.3.

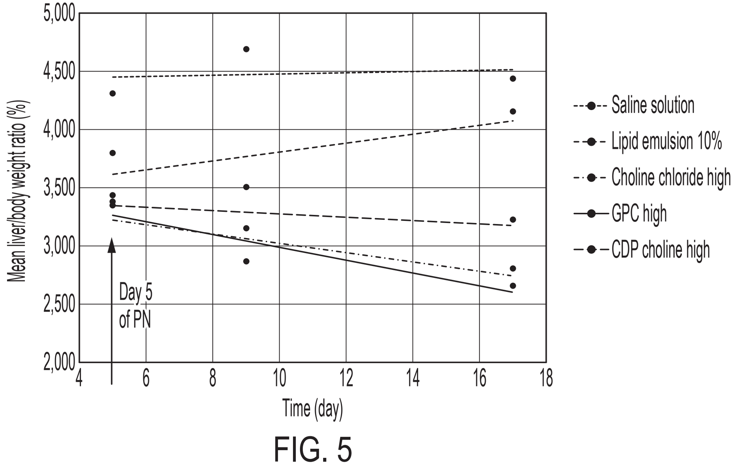

Figure 5 represents the results of the animal study as described in Example 1, wherein the influence of the parenteral administration of various lipid emulsions was determined based on the ratio of liver weight and body weight in per cent. The study animals were treated with either saline solution, LE 10% lipid emulsion ("Lipid emulsion 10%") without any choline derivative, and the same LE 10% lipid emulsion further containing 31 mmol/L of a choline derivative as shown. The expression "high" refers to a choline derivative concentration in the lipid emulsion of 31 mmol/L.

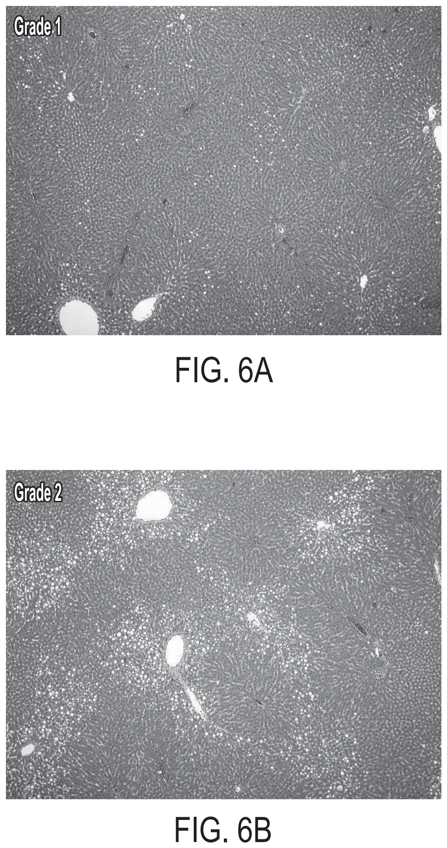

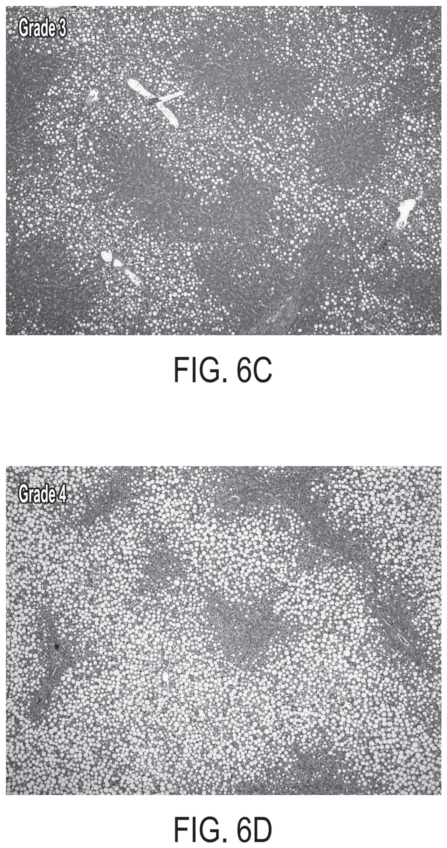

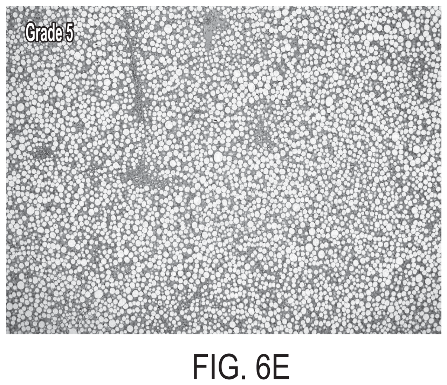

Figure 6 shows exemplary photographs of Grade 1 to 5 vacuolation determined in the histopathological analysis of the liver of study animals (see Examples 1 and 3). The histological slides shown were stained with hematoxylin-eosin. Grades were assigned according to the following scheme: Fig. 6A: GRADE 1 = Minimal/very few/very small vacuoles, scattered. Fig. 6B: GRADE 2 = Slight/few/small vacuoles, centrilobular to midzonal. Fig. 6C: GRADE 3 = Moderate/moderate number/moderate size vacuoles, centrilobular to midzonal. Fig. 6D: GRADE 4 = Marked/many/large vacuoles, centrilobular to periportal. Fig. 6E: GRADE 5 = Severe/many/large vacuoles, diffuse, all liver regions affected.

### DETAILED DESCRIPTION

Certain embodiments described herein relate generally to the field of parenteral nutrition. More particularly, some embodiments described herein relate to lipid formulations for parenteral administration, wherein the lipid formulation comprises an aqueous phase and an oil phase and the lipid formulation is present in the form of an oil-in-water emulsion. Related embodiments described herein relate to multi-chamber containers for parenteral administration, wherein the containers comprise a carbohydrate formulation in a first chamber, an amino acid formulation in a second chamber, and a lipid formulation in a third chamber.

As used herein, the term "pediatric" may refer to neonates, including premature, full term, and post-mature neonates of up to one month of age; infants of between one month and one year of age; children of between one and up to 12 years of age, and adolescents of between 13 and up to 21 years of age.

As used herein, the term "long-chain polyunsaturated fatty acid" may refer to a polyunsaturated fatty acid having at least 18 carbon atoms in the fatty acid portion of the molecule (i.e., the portion of the molecule that excludes any carbon atoms that form an ester moiety), for example, at least 20 carbon atoms, at least 22 carbon atoms, or at least 24 carbon atoms in the fatty acid portion of the molecule.

As used herein, the term "essentially free" may refer to a composition that contains no more than 5% of the specified component, for example, no more than 3%, no more than 2%, no more than 1%, no more than 0.5%, no more than 0.1%, no more than 0.05%, no more than 0.02%, no more than 0.01%, no more than 0.005%, no more than 0.002%, and/or no more than 0.001% of the specified component. For example, a composition that is "essentially free of EPA" may refer to a composition that contains no more than 5% EPA, such as no more than 3%, no more than 2%, no more than 1%, no more than 0.5%, no more than 0.1%, no more than 0.05%, no more than 0.02%, no more than 0.01%, no more than 0.005%, no more than 0.002%, and/or no more than 0.001% EPA.

The expressions "liver disease", "liver injury" and "liver damage" are interchangeably used herein. The expression refers to a condition of the liver which is defined by an increased alanine aminotransferase (ALT) activity, histologically determined vacuolation, as well as increase in the ratio of liver weight per body weight. Specifically, the expressions refer to hepatic steatosis which can be diagnosed by means of the above markers. More specifically, they refer to NAFLD. Even more specifically, they refer to NAFLD caused by choline deficiency, especially choline deficiency caused by long-term total parenteral nutrition.

### LIPID FORMULATIONS

The disclosure provides lipid formulations for parenteral administration. The lipid formulations disclosed herein include an aqueous phase and an oil phase and are present in the form of an oil-in-water emulsion. Typically, the lipid formulation includes an aqueous phase and about 5% to about 35% by weight of an oil phase based on the total weight of the lipid formulation. For example, oil phase of the lipid formulation is present in an amount about 10% to about 20%, about 10% to about 15%, about 15% to about 20%, about 12% to about 17%, about 11%, about 12%, about 13%, about 14%, about 15%, about 16%, about 17%, about 18%, and/or about 19% by weight based on the total weight of the lipid formulation.

The aqueous phase of the lipid formulation includes water. In some cases, the aqueous phase of the lipid formulation includes at least one water soluble form of choline. As used herein, "water soluble form of choline" refers to (1) water soluble salts containing the *N,N,N-*trimethylethanolammonium cation and (2) water soluble compounds containing a choline moiety, such as cytidine diphosphate choline or glycerophosphocholine. The water soluble form of choline may be selected from the group consisting of choline chloride, choline bitartrate, choline citrate, choline gluconate, choline malate, choline cytidine diphosphate choline (CDP) salt (e.g., sodium salt, potassium salt, or inner salt), and glycerophosphocholine. In a preferred embodiment, the water-soluble form of choline is present in a concentration of from 0.1 g to 12 g of choline equivalent per liter of lipid emulsion, for example, from 0.2 g to 10 g of choline equivalent per liter of lipid emulsion, from 3 g to 6 g of choline equivalent per liter of lipid emulsion, from 0.5 to 4 g of choline equivalent per liter of lipid emulsion, from 0.1 g to 0.5 g of choline equivalent per liter of lipid emulsion, from 0.5 g to 1 g of choline equivalent per liter of lipid emulsion, from 1 g to 2 g of choline equivalent per liter of lipid emulsion, from 2 g to 3 g of choline equivalent per liter of lipid emulsion, from 3 g to 4 g of choline equivalent per liter of lipid emulsion, from 4 g to 5 g of choline equivalent per liter of lipid emulsion, from 5 g to 6 g of choline equivalent per liter of lipid emulsion, from 6 g to 7 g of choline equivalent per liter of lipid emulsion, from 7 g to 8 g of choline equivalent per liter of lipid emulsion, from 8 g to 9 g of choline equivalent per liter of lipid emulsion, from 9 g to 10 g of choline equivalent per liter of lipid emulsion, from 10 g to 11 g of choline equivalent per liter of lipid emulsion, and/or from 11 g to 12 g of choline equivalent per liter of lipid emulsion. In a further preferred embodiment, the water soluble form of choline is glycerophosphocholine in a concentration of from 0.1 g to 15.0 g per liter of lipid emulsion, for example, from 0.1 g to 6.0 g per liter of lipid emulsion, from 0.1 g to 2.0 g per liter of lipid emulsion, from 0.2 g to 0.9 g per liter of lipid emulsion, from 0.5 to 6.0 g per liter of lipid emulsion, from 0.5 g to 5.0 g per liter of lipid emulsion, from 0.5 g to 4.0 g per liter of lipid emulsion, from 0.5 g to 3.0 g per liter of lipid emulsion, from 1.0 g to 12.0 g per liter of lipid emulsion, from 1.0 g to 10.0 g per liter of lipid emulsion, from 1.0 g to 5.0 g per liter of lipid emulsion, from 2.0 g to 12.0 g per liter of lipid emulsion, from 2.0 g to 9.0 g per liter lipid emulsion, from 2.0 g to 4.0 g per liter of lipid emulsion, from 4.0 g to 11.0 g per liter of lipid emulsion, from 4.0 g to 10.0 g per liter of lipid emulsion, or from 5.0 g to 12.0 g per liter of lipid emulsion.

The oil phase of the lipid formulation includes polyunsaturated fatty acids, such as long-chain polyunsaturated fatty acids, which may be present as the free acid, as an ionized or salt form of the free acid, and/or in ester form. Suitable esters of the polyunsaturated fatty acids/long-chain polyunsaturated fatty acids include, but are not limited to, alkyl esters (e.g., methyl esters, ethyl esters, propyl esters, or combinations thereof) and triglyceride esters. In some cases, the long-chain polyunsaturated fatty acid has a structure R(C=O)OR', wherein R is an alkenyl group having at least 17 carbon atoms, at least 19 carbon atoms, at least 21 carbon atoms, or at least 23 carbon atoms, and R' is absent, H, a counter ion, an alkyl group (e.g., methyl, ethyl, or propyl), or a glyceryl group (e.g., R(C=O)OR' is a monoglyceride, a diglyceride, or a triglyceride). Polyunsaturated fatty acids for use in the lipid formulations disclosed herein include, but are not limited to, linoleic acid (LA), arachidonic acid (ARA), α-linolenic acid (ALA), eicosapentaenoic acid (EPA), docosahexaenoic acid (DHA), stearidonic acid (SDA), γ-linolenic acid (GLA), dihomo-γ-linolenic acid (DPA), and docosapentaenoic acid (DPA), particularly, DHA, ARA, and EPA, each of which may be present in free acid form, ionized or salt form, alkyl ester form, and/or triglyceride form. In some cases, the polyunsaturated fatty acids and/or long-chain fatty acids are present in triglyceride form.

In some cases, the polyunsaturated fatty acids for the lipid formulations (e.g., the long-chain polyunsaturated fatty acids), are obtained from a single source. In some cases, the single source is an extract of microalgae. Suitable micro-algal sources include, but are not limited to, *Crypthecodinium cohnii* and *Schizochytrium sp.* In some cases, *Crypthecodinium cohnii* or *Schizochytrium sp.* is the single source of the long-chain polyunsaturated fatty acid DHA. In some cases, the amount of DHA in an oil isolated from *Schizochytrium sp.* will be about 250 to about 700 mg/g of oil, such as about 300 to about 600 mg/g, about 350 to about 550 mg/g, and/or about 400 to about 500 mg/g of oil.

When the lipid formulation includes *Schizochytrium sp.* as the single source of polyunsaturated fatty acids, the lipid formulation includes DHA in relatively greater amount that EPA or ARA. For example, when *Schizochytrium sp.* is the single source of the polyunsaturated fatty acids in the lipid formulation, the ratio of DHA to EPA in the lipid formulation is typically at least 10:1 (w/w), for example about 10:1 to about 1000:1 (w/w), about 20:1 to about 1000:1 (w/w), about 50:1 to about 1000:1 (w/w), about 100:1 to about 1000:1 (w/w), about 10:1 to about 200:1 (w/w), about 20:1 to about 150:1 (w/w), at least about 50:1 (w/w), at least about 100:1 (w/w), at least about 500:1 (w/w), and/or at least about 1000:1 (w/w). Further, when *Schizochytrium sp.* is the single source of the polyunsaturated fatty acids in the lipid formulation, the ratio of DHA to ARA in the lipid formulation is typically at least 20:1 (w/w), for example about 20:1 to about 1000:1 (w/w), about 50:1 to about 1000:1 (w/w), about 100:1 to about 1000:1 (w/w), at least about 50:1 (w/w), at least about 100:1 (w/w), at least about 500:1 (w/w), and/or at least about 1000:1 (w/w).

In some cases, the oil phase includes DHA obtained from a single cell source, wherein the single cell source is an extract of microalgae. In some cases, DHA is present in a concentration of from 0.1 g to 5.0 g per 100 g of oil phase, for example, 0.25 g to 3.0 g per 100 g of oil phase, and/or 1.5 g to 2.5 g per 100 g of oil phase. In some cases, the lipid formulation comprises eicosapentaenoic acid (EPA) and the ratio of DHA to EPA in the lipid formulation is 10:1 to 1000:1 (w/w), for example, 10:1 to 200:1 (w/w) and/or 20:1 to 150:1 (w/w).

In some cases, the lipid formulation is essentially free of EPA.

In some cases, the lipid formulation is essentially free of water soluble forms of choline, such as water soluble salts containing the N,N,N-trimethylethanolammonium cation and/or water soluble compounds containing a choline moiety.

In some cases, the lipid formulation is essentially free of ARA.

In some cases, the oil phase includes a blend of oils derived from the microalgae *Schizochytrium sp.* and the fungi *Mortierella alpina.* The oils in such a blend include, but are not limited to, myristic acid (C14:0), palmitic acid (C16:0), palmitoleic acid (C16:1), stearic acid (C18:0), oleic acid (C18:1), linoleic acid (C18:2 n-6), gamma-linolenic acid (C18:3, n-6), alphalinolenic acid (C18:3, n-3), arachidic acid (C20:0), ARA (C20:4, n-6), EPA (C20:5, n-3), behenic acid (C22:0), DPA (C22:5, n-3), DHA (C22:6, n-3), and lignoceric acid (C24:0). In some cases, the blend includes ARA, DHA, and EPA.

In some cases, ARA is included in the lipid formulation. When ARA is added to the lipid formulation, the formulation typically contains a greater amount of ARA than when *Schizochytrium sp.* is the single source of the polyunsaturated fatty acids in the lipid formulation. In some cases, the ratio of DHA to ARA in the lipid formulation is about 10:1 to about 1:5 (w/w), for example, about 5:1 to about 1:5 (w/w), about 3:1 to 1:3 (w/w), about 1:1 to about 1:3 (w/w), about 1:1.5 to about 1:2.5 (w/w), such as about 1:2 (w/w). Further, when ARA is added to the lipid formulation, the ratio of DHA to EPA in the lipid formulation can be at least 10:1 (w/w), for example about 10:1 to about 1000:1 (w/w), about 20:1 to about 1000:1 (w/w), about 50:1 to about 1000:1 (w/w), about 100:1 to about 1000:1 (w/w), about 10:1 to about 200:1 (w/w), about 20:1 to about 150:1 (w/w), at least about 50:1 (w/w), at least about 100:1 (w/w), at least about 500:1 (w/w), and/or at least about 1000:1 (w/w).

In some cases, the oil phase includes ARA obtained from a single cell source, wherein the single cell source is fungi, such as an extract of fungi. In a preferred embodiment, the fungus is *Mortierella alpina.* In some cases, ARA is present in a concentration of from 0.1 g to 15 g per 100 g of oil phase, for example, from 1.5 g to 7.5 g per 100 g of oil phase.

The lipid formulations disclosed herein typically include reduced levels of phytosterols. In particular, the oils used in the lipid formulations have been depleted in phytosterols, typically by an amount of at least 25% of the total amount of phytosterols initially present in the oil, for example, at least 40%, at least 50%, at least 60%, and/or at least 75% depleted. In some cases, the lipid formulations include 70 mg phytosterols or less per 100 g of oil phase, for example, about 15 mg to about 35 mg phytosterols per 100 g of oil phase, and/or about 25 mg phytosterols or less per 100 g of oil phase. Removal or depletion of phytosterols can be carried out by, for example, short path distillation, active charcoal treatment following by filtration, supercritical CO₂ chromatography, or chromatographic purification.

The lipid formulations disclosed herein may further include saturated, monounsaturated, and polyunsaturated fatty acids. In some cases, these fatty acids are present in a concentration of from 95 g to 99.1 g per 100 g of oil phase, for example, 97 g to 99.75 per 100 g of oil phase, and/or 97.5 g to 98.5 g per 100 g of oil phase. In some cases, these fatty acids may be obtained from animal and/or plant sources. In some cases, these fatty acids may be present mainly as triglycerides.

The lipid formulations disclosed herein may further include additional components, such as surfactants (also referred to as emulsifiers), co-surfactants, isotonic agents, pH adjusters, and antioxidants. Generally, surfactants are added to stabilize emulsions by reducing the interfacial tension between the oil phase and the aqueous phase. Surfactants typically include a hydrophobic part and a hydrophilic part, and the amount of surfactant/emulsifier included in the formulations is determined based on the amount that is needed to achieve a desired level of stabilization of the emulsion. Typically, the amount of surfactant in the lipid formulation is about 0.01 % to about 3% by weight based on the total weight of the lipid formulation, for example, about 0.01 % to about 2.5%, about 0.01 % to about 2.3%, about 0.02 % to about 2.2%, about 0.02 % to about 2.1%, about 0.02 % to about 2%, about 0.05% to about 1.8%, about 0.1% to about 1.6%, about 0.5% to about 1.5%, about 0.8% to about 1.4%, about 0.9% to about 1.3%, about 1% to about 1.2%, and/or about 1.2% by weight. Suitable surfactants and co-surfactants include surfactants that are approved for parenteral use, and include, but are not limited to, phospholipids (e.g., egg phosphatide and soy lecithin), oleate salts, and combinations thereof. An exemplary surfactant is lecithin, including both natural and synthetic lecithin, such as lecithins derived from egg, corn or soybean or mixtures thereof. In some cases, lecithin is included in an amount of about 1.2% based on the total weight of the lipid formulation. In some cases, the lipid emulsion formulation includes a co-surfactant. Typically, the amount of co-surfactant in the lipid formulation is less than the amount of surfactant, and typically the amount of co-surfactant in the formulation is about 0.001% to about 0.6% by weight based on the total weight of the lipid formulation, for example, about 0.001% to about 0.55%, about 0.001% to about 0.525%, about 0.001% to about 0.5%, about 0.005% to about 0.5%, about 0.01% to about 0.4%, about 0.02% to about 0.3%, about 0.03% to about 0.2%, about 0.04% to about 0.1%, and/or about 0.05% to about 0.08%. An exemplary co-surfactant is oleate, such as sodium oleate. In some cases, the lipid formulation includes lecithin and oleate as surfactant and co-surfactant, for example, an in amount of 1.2% lecithin and 0.03% oleate. In some cases, sodium oleate is included in an amount of about 0.03% by weight based on the total weight of the lipid formulation.

Isotonic agent can be added to the lipid formulations to adjust the osmolarity of the lipid emulsion formulation to a desired level, such as a physiologically acceptable level. Suitable isotonic agents include, but are not limited to, glycerol. Typically, the lipid emulsion formulation has an osmolarity of about 180 to about 300 milliosmols/liter, such as about 190 to about 280 milliosmols/liter, and/or about 200 to about 250 milliosmols/liter. In some cases, the lipid emulsion formulation includes an isotonic agent in an amount of about 1% to about 10% by weight based on the total weight of the lipid formulation, such as about 1% to about 5%, about 1% to about 4%, and/or about 2% to about 3%. In some cases, the lipid emulsion formulation includes about 2% to about 3% by weight of glycerol.

pH modifiers can be added to the lipid formulations to adjust the pH to a desired level, such as a physiologically acceptable pH for parenteral use. Suitable pH modifiers include, but are not limited to sodium hydroxide and hydrochloric acid. Typically, the lipid emulsion formulation has a pH of about 6 to about 9, such as about 6.1 to about 8.9, about 6.2 to about 8.8, about 6.3 to about 8.7, about 6.4 to about 8.6, about 6.5 to about 8.5, about 6.6 to about 8.4, about 6.7 to about 8.3, about 6.8 to about 8.2, about 6.9 to about 8.1, about 7 to about 8, about 7.1 to about 7.9, about 7.2 to about 7.8, about 7.3 to about 7.7, about 7.4 to about 7.6, about 7, about 7.5, and/or about 8.

The lipid formulations may further include antioxidants. Suitable antioxidants may be pharmaceutically acceptable antioxidants and include, but are not limited to, tocopherols (e.g., gamma tocopherol, delta tocopherol, alpha tocopherol), ascorbyl palmitate, or combinations thereof. In some cases, the lipid emulsion formulation includes an antioxidant in an amount of about 0 to about 200 mg/L, for example, about 10 to about 200 mg/L, about 40 to about 150 mg/L, about 50 to about 120 mg/L, about 75 to about 100 mg/L antioxidant, such as vitamin E.

The aqueous (or water) phase of all intravenous lipid emulsions must conform to the pharmacopeial requirements that make it suitable for injection, that is, the water must be sterile water for injection.

Also included in the disclosure are lipid emulsions wherein the choline source (e.g., GPC), if present, is added, e.g. through a medical port, to a ready-to-use lipid emulsion or a reconstituted solution before administration to a patient. The choline source (e.g., GPC) can be provided in the form of a suitable solution, for example in a vial or a suitable flexible or rigid container, or can be provided in lyophilized form, for example in a glass vial, wherein the lyophilized choline source (e.g., GPC) is dissolved in a suitable solvent before adding it to the lipid emulsion or reconstituted solution. The choline source (e.g., GPC) can be present as the single active component in such solution or lyophilisate for addition to a lipid emulsion or reconstituted solution, or can be present in combination with at least one further active component such as, for example, vitamins, trace elements, DHA, EPA and/or ARA. Therefore, according to another embodiment of the invention, a method of providing a choline source (e.g., GPC) to a patient is provided, wherein the choline source (e.g., GPC) is added to a ready-to-use lipid emulsion or a reconstituted solution from a MCB providing for formulations for parenteral nutrition before administration.

A first lipid formulation according to the disclosure includes an oil phase comprising docosahexaenoic acid (DHA) obtained from a single cell source, wherein the single cell source is an extract of microalgae and wherein DHA is present in a concentration of from 0.1 g to 5.0 g per 100 g of oil phase. Further, the oils provided for the first lipid formulation have been depleted in phytosterols such that the lipid formulation includes about 70 mg phytosterols or less per 100 g of oil phase. Additionally, the first lipid formulation is essentially free of water soluble forms of choline and/or arachidonic acid (ARA). As discussed in more detail herein, the first lipid formulation includes an aqueous phase and about 5% to about 35% by weight of an oil phase based on the total weight of the lipid formulation and the lipid formulation is present in the form of an oil-in-water emulsion.

A second lipid formulation according to the disclosure includes at least one pharmaceutically acceptable water soluble form of choline and an oil phase comprising docosahexaenoic acid (DHA). Further, the oils provided for the second lipid formulation have been depleted in phytosterols such that the lipid formulation includes about 70 mg phytosterols or less per 100 g of oil phase. Additionally, lipid formulation is essentially free of arachidonic acid (ARA). As discussed in more detail herein, the second lipid formulation comprises an aqueous phase and about 5% to about 35% by weight of an oil phase based on the total weight of the lipid formulation and the lipid formulation is present in the form of an oil-in-water emulsion.

A third lipid formulation according to the disclosure includes at least one pharmaceutically acceptable water soluble form of choline, and an oil phase comprising docosahexaenoic acid (DHA) and arachidonic acid (ARA). The third lipid formulation has a ratio of DHA to ARA in the lipid formulation is 10:1 to 1:5 (w/w). Further, the oils provided for the third lipid formulation have been depleted in phytosterols such that the lipid formulation includes about 70 mg phytosterols or less per 100 g of oil phase. As discussed in more detail herein, the third lipid formulation comprises an aqueous phase and about 5% to about 35% by weight of an oil phase based on the total weight of the lipid formulation and the lipid formulation is present in the form of an oil-in-water emulsion.

In some cases, an oil phase according to the disclosure comprises omega-3 fatty acids consisting of DHA and a low amount of EPA, and omega-6 fatty acids such as ARA.

The lipid emulsions disclosed herein can be prepared according to generally known processes (see, for example, Hippalgaonkar et al, AAPS PharmSciTech 2010, 11(4), 1526-1540). Generally, water soluble and oil-soluble ingredients are dissolved in the aqueous phase and oil phase, respectively. Accordingly, the choline source, if present, is dissolved in the aqueous phase. Emulsifiers, such as phosphatides, can be dispersed in either oil or aqueous phase. Both phases are adequately heated and stirred to disperse or dissolve the ingredients. The lipid phase is then generally added to the aqueous phase under controlled temperature and agitation (using high-shear mixers) to form a homogenously dispersed coarse or pre-emulsion. Pre-emulsions with a droplet size smaller than 20 µm generally produces unimodal and physically stable fine emulsions. The pre-emulsion is then homogenized (using a microfluidizer or a high-pressure homogenizer) at optimized pressure, temperature, and number of cycles to further reduce the droplet size and form a fine emulsion. Factors such as type and concentration of oil phase and surfactants, operating temperature, pressure, number of cycles, etc. can influence the mean droplet size during high-pressure homogenization and microfluidization. Throughout the shelf-life of an emulsion, the mean droplet size and PFAT5 (volume-weighted percentage of fat globules ≥5 µm) of an injectable fine emulsion should be ≤500 nm and ≤0.05%, respectively. The pH of the resulting fine emulsion is then adjusted to the desired value and the emulsion is filtered through 1-5 µm filters. The fine emulsions are then transferred into suitable containers. Plastic containers which are permeable to oxygen and/or contain oil-soluble plasticizers and usually avoided. The entire process (filtration/coarse and fine emulsion preparation) is usually carried out under nitrogen atmosphere whenever possible and especially in cases where the excipients and specific components of the lipid emulsion are sensitive to oxidation. Sterilization of the lipid formulations can be achieved by terminal heat sterilization or by aseptic filtration. Terminal sterilization generally provides greater assurance of sterility of the final product. However, if the components of the emulsions are heat labile, sterile filtration can be used. Sterilization by filtration requires the emulsion droplet size to be below 200 nm. Alternatively, aseptic processing may be employed. However, this process is relatively equipment and labor intensive and requires additional process validation data and justification during regulatory submissions.

Accordingly, lipid emulsions according to the invention can be prepared by the following steps comprising:
a. Separately heating up the oil phase and the aqueous phase to a temperature of from about 70°C to about 80°C under agitation;
b. Adding a choline source such as glycerophosphocholine to the aqueous phase;
c. Preparing the pre-emulsion by transferring the oil phase to the aqueous phase under agitation;
d. Homogenizing the pre-emulsion at a temperature of from about 40°C to 60°C under pressure;
e. Optionally adding water to adjust the required volume and concentrations;
f. Optionally adjusting the pH to a range of from about 7.8 to 8.8; and
g. Optionally sterilizing the lipid emulsion.

Sterilization can be done by methods known in the art, such as, for example, by heat. Usually, steps (a) through (d) will be performed in the presence of an inert gas, such as N₂, for avoiding any oxidation reactions. The pressure used for homogenization of the pre-emulsion can vary over a broad range. Generally, it will be in a range of from 100 to 1300 bar, from 200 to 1000 bar, from 300 to 800 bar, or from 400 to 1100 bar.

### AMINO ACID FORMULATIONS

The amino acid formulation includes a sterile, aqueous solution of one or more amino acids and one or more electrolytes. Typically, the amino acid formulation includes about 2 grams to about 10 grams of amino acids per 100 mL of amino acid formulation, such as about 3 grams to about 9 grams, about 4 grams to about 8 grams, and/or about 5 grams to about 7 grams per 100 mL of amino acid formulation. The amino acid formulation generally includes isoleucine, leucine, valine, lysine, methionine, phenylalanine, threonine, tryptophan, arginine, histidine, alanine, aspartic acid, cysteine, glutamic acid, glycine, proline, serine, tyrosine, ornithine, and taurine. Further, the content of tyrosine can be increased by adding, for example, a glycyl-tyrosine dipeptide or acetyl-tyrosine (Ac-Tyr). Typically, however, the glycyl-tyrosine dipeptide has improved pharmacokinetics compared to Ac-Tyr, which is more rapidly eliminated by the kidney, resulting in diminished release of tyrosine in the blood.

The amino acid formulation may further include electrolytes such as sodium, potassium, calcium, magnesium, and/or phosphate ions. For example, the amino acid formulation can include from about 0.1 mmol to about 10 mmol of sodium (e.g., about 3.75 mmol to about 10 mmol of sodium), from about 0.1 mmol to about 10 mmol of potassium (e.g., about 3.75 mmol to about 6.90 mmol of potassium), from about 0.05 mmol to about 1.0 mmol of magnesium (e.g., about 0.05 mmol to about 0.11 mmol and/or about 0.38 mmol to about 0.65 mmol of magnesium), from about 0.1 mmol to about 10 mmol of calcium (e.g., about 1.13 mmol to about 5.10 mmol of calcium), from about 0.1 mmol to about 10 mmol of phosphate (e.g., about 0.94 mmol to about 5.10 mmol of phosphate) and not more than 10 mmol of chloride (e.g., not more than 5.6 mmol of chloride) per 100 mL of amino acid formulation. When calcium and phosphorus are present together in the same heat-sterilized solution, insoluble calcium phosphate precipitation can occur. Using an organic salt of phosphorus such as sodium glycerophosphate 5·H₂O or calcium glycerophosphate, calcium and phosphate amounts may be increased without solubility issues and without providing excess sodium or chloride. In the amino acid formulation, sodium may be provided in the form of sodium chloride, calcium may be provided in the form of calcium chloride 2·H₂O or calcium gluconate, magnesium may be provided in the form of magnesium acetate 4·H₂O or magnesium chloride, and potassium may be provided in the form of potassium acetate.

The amino acid formulation may further include a water-soluble form of choline selected from the group consisting of choline chloride, choline bitartrate, choline citrate, choline gluconate, choline malate, choline cytidine diphosphate choline (CDP) salt and glycerophosphocholine. In a preferred embodiment, the water-soluble form of choline is present in a concentration of from 20 mg to 2000 mg choline equivalent per liter of the reconstituted multi-chamber container, for example, from 30 mg to 1000 mg choline equivalent per liter of the reconstituted multi-chamber container.

### CARBOHYDRATE FORMULATIONS

The carbohydrate formulations provide a supply of calories, typically in the form of glucose. In particular, the carbohydrate formulation provides an amount of carbohydrate sufficient to avoid adverse effects such as hyperglycemia that has been observed in patients receiving parenteral nutrition. Typically, the carbohydrate formulation includes about 20 to 50 grams of glucose per 100 mL of carbohydrate formulation.

The carbohydrate formulation may further include a water-soluble form of choline selected from the group consisting of choline chloride, choline bitartrate, choline citrate, choline gluconate, choline malate, choline cytidine diphosphate choline (CDP) salt and glycerophosphocholine. In a preferred embodiment, the water-soluble form of choline is present in a concentration of from 20 mg to 2000 mg choline equivalent per liter of the reconstituted multi-chamber container, for example, from 30 mg to 1000 mg choline equivalent per liter of the reconstituted multi-chamber container.

### MULTI-CHAMBER CONTAINERS

The disclosure provides a multi-chamber container for parenteral administration of nutritional formulations. For example, the container may be in the form of a bag having multiple compartments or chambers. The container, such as a bag, includes at least two chambers, such as three, four, or five chambers, and in one preferred embodiment, three chambers. Suitable containers, including bags, typically are sterile, non-pyrogenic, single-use, and/or ready-to-use. The multi-chamber containers are particularly useful for holding a pediatric and/or neonatal parenteral nutrition product and generally provide a carbohydrate formulation as disclosed herein in the first chamber, an amino acid formulation as disclosed herein in a second chamber, and a lipid formulation as disclosed herein in a third chamber of the container.

The multi-chamber container, such as a three-chamber bag, may include vertical chambers. Suitable multi-chamber containers are disclosed in U.S. Patent Publication No. 2007/0092579, which is incorporated by reference in its entirety. For example, the multi-chamber container may be configured as a bag that includes three adjacent chambers or compartments. If desired, frangible barriers or openable seals (e.g., peel seals or frangible seals) are used to separate the chambers of the multi-chamber container. The openable seals permit formulations to be separately stored and admixed just prior to administration thereby allowing storage in a single container of formulations which should not be stored as an admixture for an extended period of time. Opening of the seals allows communication between the chambers and mixing of the contents of the respective chambers. The outside seals of the multi-chamber container are strong seals that do not open under the fluid pressure supplied to open the weaker peel seals or frangible seals between the chambers.

The multi-chamber container may be provided with instructions explaining a desired order with which to open the peel seals, so that constituent fluids are mixed in a desired order. The unsealing strengths of the two or more peel seals may be varied to promote the opening of the seals in the desired order. For example, the unsealing strength of the peel seal to be opened first may be 1/3 to 1/2 of the unsealing strength required to open the peel seal to be opened second.

FIG. 1 illustrates one embodiment of a multiple chamber container of the present invention. Preferably, the container 10 which is configured as a bag includes three adjacent chambers or chambers 12, 14, and 16. Chamber 12 is located at a lateral or side end 18 and chamber 16 is located at an opposite lateral or side end 20. The three chambers 12, 14, and 16 are preferably designed to hold aqueous solutions and/or lipid emulsions. Preferably, frangible barriers or openable seals 22 and 24 are used to separate the chambers. Container 10 preferably includes ports 26, 28, and 30 to provide communication with chambers 12, 14, and 16 respectively. Container 10 also preferably provides a hanger portion 36 which in the embodiment shown in FIG. 1 is a flap having a centrally located hole 38 for hanging the container. The flap 36 defines a border 40 of the upper end of all the chambers 12, 14, and 16.

### METHODS OF USE

The disclosure provides methods of treating patients who require parenteral nutrition when oral and enteral nutrition is not possible, insufficient or contraindicated. The methods involve using the multi-chamber containers and lipid formulations disclosed herein. In particular, the methods involve parenterally administering the contents of a multi-chamber container and/or lipid formulations as disclosed herein to a patient. In a preferred embodiment, the patients are pediatric patients.

In some cases, the lipid emulsions and compositions comprising same disclosed herein are used for avoiding and/or treating hepatic steatosis. Hepatic steatosis is defined as intrahepatic fat of at least 5% of liver weight. Simple accumulation of triacylglycerols in the liver could be hepatoprotective; however, prolonged hepatic lipid storage may lead to liver metabolic dysfunction, inflammation, and advanced forms of nonalcoholic fatty liver disease (NAFLD). NAFLD includes a spectrum of disease from simple steatosis to nonalcoholic steatohepatitis (NASH), which can progress to cirrhosis and hepatocellular carcinoma. Histologically, NASH is defined by the presence of macrovesicular steatosis, lobular inflammation, and hepatocellular ballooning (Kneeman, Secondary Causes of nonalcoholic fatty liver disease, Therap Adv Gastroenterol 2012; 5: 199-207). Long-term total parenteral nutrition (TPN) can be one of many causes for NAFLD. TPN may lead to the depletion of carnitine, a compound necessary for the transfer of free fatty acids from the hepatic cytoplasm into the mitochondria for beta-oxidation. As mentioned before, the cytosolic concentration of choline also decreases, promoting lipid storage in hepatocytes. Such lipid storage is connected to the fact that predominant histologic findings are steatosis, intrahepatic cholestasis, and ductular reduplication. Accordingly, the effect of choline supplementation in TPN can also be determined by validating the histological features of the liver. Such histological analysis allows assessment of the degree of steatosis by analyzing the extent of vacuolation. Example 3 provides for the results of such histopathological investigations done in an animal study. It is shown there that liver damage due to methionine and choline deficient diet followed by parenteral nutrition can be effectively addressed by the presence of glycerophosphocholine in the PN solution. It also provides for the evidence that GPC provides for a significantly better effect than other choline derivatives, specifically choline chloride and CDP-choline. The additional presence of DHA and ARA seem to have an additional, synergistic benefit.

Serum alanine aminotransferase (ALT) assays are the most common laboratory tests for the detection of liver diseases. Because the enzyme concentration in a population forms a continuous distribution, the cut-off concentration that discriminates between healthy and diseased livers is not clearly defined. However, the upper normal limit of serum alanine aminotransferase is set on average at 40 IU/l, ranging from 30-50 IU/1 (Kim et al. BMJ 2004, 328:983). Said upper limit is used as a normal serum alanine aminotransferase (referred herein also as "aminotransferase" or "ALT") concentration in the context of the present invention. Kim et al. also describe that the association between the aminotransferase concentration and mortality from liver disease is significant. Therefore, serum aminotransferase concentration was used in the context of the present invention to determine the efficacy of glycerophosphocholine for the treatment of liver damage which can be caused, for example, by parenteral nutrition in comparison to lipid emulsion not containing any choline, but also in comparison with other choline sources, including choline chloride and CDP-choline. Figures 1 and 2 demonstrate the significantly better effect of GPC on lowering ALT concentrations.

ALT activity is determined by a modification recommended by the International Federation of Clinical Chemistry (IFCC), see also Example 2. ALT catalyzes the reaction of alpha-ketoglutarate with L-alanine to form L-glutamate and pyruvate. Under the action of LDH, pyruvate converts to lactate, and NADH is converted to NAD. The decrease in absorbance of NADH, measured at 340 nm (secondary wavelength is 700 nm), is directly proportional to the serum activity of ALT. It is a kinetic reaction. In general, serum is used for analysis. The serum is separated from the cells within 2 hours of collection and stored at -70° C until assayed. Monitoring the ALT activity over time in an animal model is used herein as one straightforward and conclusive way to determine the efficacy of GPC in treating liver disease, specifically liver damage caused by a choline-deficient diet (Examples 1 and 2, Figures 2 and 3).

Plasma-free choline concentration can be determined by high-pressure liquid chromatography (HPLC) and gas chromatography-mass spectrometry according to protocols known in the art (Pomfret et al.: Measurement of choline and choline metabolite concentrations using high pressure liquid chromatography and gas chromatography-mass spectrometry. Anal Biochem. 1989; 180: 85-90).

Methods of depleting phytosterol from an oil, specifically from a plant derived oil, such as olive or soybean oils, are known. For example, phytosterols can be removed as described in Ostlund et al., Am J Clin Nutr 2002, 75, 1000-1004, or as described in US 6303803 B1.

### EXAMPLES

### Example 1: Animal model

Animal studies were performed in order to identify and confirm the most effective choline derivative for use in parenteral nutrition solutions, specifically in lipid emulsions, for avoiding and/or treating and ameliorating liver damage, specifically liver steatosis. The study further served to identify any potential metabolic interactions between the choline derivate which was found to be the most effective and polyunsaturated fatty acids, specifically of DHA and ARA.

The effects of GPC as well as of the combination of DHA, ARA and GPC were investigated in a sick animal model, wherein male Sprague-Dawley rats with steatosis, induced by choline and methionine deficient (MCD) diet (SSNIFF MCD pelleted choline/methionine deficient diet (SSNIFF Spezialdiäten GmbH, Soest, Germany, Product No. E15653-94)) were kept on parenteral nutrition. Steatosis was confirmed in the respective groups by histopathology (Example 3).

Parenteral nutrition started after 14 days under MCD diet. First measurements were done after 5 days of parenteral nutrition, followed by measurements after 9 and 17 days. The daily lipid uptake was 2.31 g/kg/d. The rats were split into several groups as set forth below in Table I.

Two groups served as control groups, wherein a first group was treated with saline solution (0.90% w/v of NaCl in water for injection). A second group received a lipid emulsion ("LE 10%") containing refined olive oil (80.0 g/L) and refined soybean oil (20.0 g/L) in water for injection, pH 6-8; LE 10% further contained egg lecithin (6.0 g/L), glycerol (11.25 g/L) and sodium oleate (0.15 g/L). LE 10% contained 100 g/L lipids. In certain experiments the lipid emulsion "LE" was used in different concentrations, for example as "LE 13%", or "LE 20%" which indicates that the above concentrations were accordingly higher.

Various choline derivatives selected from the group consisting of choline chloride, glycerophosphocholine (GPC) and cytidine diphosphate-choline (CDP-choline) were added in two concentrations (low/high) to LE 10%, respectively, see Table I (Step#2). "Low" refers to a concentration of 15 mmol/L lipid emulsion, "high" refers to a concentration of 31 mmol/L of lipid emulsion. Choline chloride (2-Hydroxyethyl)trimethylammonium chloride), Pharmaceutical Secondary Standard, was purchased from Sigma-Aldrich (Merck KGaA, Germany). CDP-choline (prepared by fermentation) was from TCI Europe N.V. (Antwerp, Belgium). Glycerophosphocholine, prepared from egg phosphatide by saponification, was supplied by Lipoid GmbH, Germany. DHA (from algae, Schizochytrium sp.) and ARA (from fungi, Mortierella alpina) were purchased from BASF AG, Germany. Vitamin E was purchased from ADM (IL, U.S.A.)

The choline derivative supplemented "LE" lipid emulsions were prepared as follows. In a first step, raw phases are heated up. In case of the oil phase, soybean and olive oils and egg phosphatide as well as DHA and ARA oils and vitamin E in case of Step#3 experiments are heated in an inox beaker to 75°C under N₂ protection while continuously agitating using an Ultra Turrax. The aqueous phase was prepared by mixing glycerol, sodium oleate and Milli-Q water and constantly heated in an inox beaker to about 75°C under N₂ protection. Where needed, the choline derivative is added under constant agitation. In a second step, the pre-emulsion is prepared by adding the oily phase to the aqueous phase using a peristaltic pump under continuous agitation and then transferred to a high shear inline disperser (e.g. Dispax Reactor^{®} DR). The receiving vessel is closed and flushed with nitrogen. In a third step the pre-emulsion is homogenized by passing it several times through a high-pressure homogenizer at 600 bar and a temperature of about 50 °C, under a N₂ atmosphere.

The emulsion is then adjusted to the required volume (2L) with Milli-Q water and cooled down to a temperature of approximately 18 to 28°C, preferably 20°C to 25°C. The pH is adjusted to a range of from 7.8 to 8.8, preferably to about 8.3 to 8.8 with 0.1N NaOH. The emulsion is filtered with a 4.5 µm filter and transferred in 100 mL bags, which are then overpouched, making use of an oxygen absorber/indicator, for later use. The lipid emulsion is then autoclaved at 121°C to achieve a F0 of at least 15 minutes.

**Table I: Study Groups and Experimental Setup: Standard "LE" lipid emulsion was used for the experiments wherein certain amounts of a choline source were added to the lipid emulsion. This test setup which is focused on the effect of GPC as such is otherwise designated as "Step#2")**

| **Group** | **Lipid Emulsion/Solution** | **Choline Equivalent [mmol/L]** | **Dose [mmol/kg/day]** |
|---|---|---|---|
| **Control 1** | Saline Solution | - | - |
| **Control 2** | LE 10% | - | - |
| **Efficacy** | LE 10% | Choline Chloride [15] | 0,38* |
| | | Choline Chloride [31] | 0,77* |
| **Efficacy** | LE 10% | Glycerophosphocholine [15] | 0,38* |
| | | Glycerophosphocholine [31] | 0,77* |
| **Efficacy** | LE 10% | CDP-choline [15] | 0,38* |
| | | CDP-choline [31] | 0,77* |

**Table II: Study Groups and Experimental Setup: Phytosterol depleted "LE" lipid emulsion as described above was used for experiments, wherein GPC as well as DHA and ARA were added as indicated. This test setup is otherwise designated as "Step#3").**

| **Group** | **Lipid emulsion/Solution** | **Choline Source [mmol/L]** |
|---|---|---|
| **Control** | Saline solution | - |
| **Control** | LE 13%, phytosterol depleted, + DHA and ARA + vitamin E supplementation (40 µg/mL LE) | none |
| **Efficacy** | LE 13%, phytosterol depleted, + vitamin E supplementation (40 µg/mL LE) | Glycerophosphocholine [40 mmol/L] |
| **Efficacy** | LE 13%, phytosterol depleted + DHA and ARA + vitamin E supplementation (40 µg/mL LE) | Glycerophosphocholine [40 mmol/L] |

### Example 2: Aminotransferase (ALT) development

For assessing the effect of GPC on the liver status of the animals tested as described in Example 1, the aminotransferase (ALT) activity in the plasma of tested animals was tested after 5 and 17 days of parenteral nutrition both in a Step#2 and Step#3 setup. As described before, liver damage (cholestasis) may be determined by increased aminotransferase levels. ALT activity was determined with an ADVIA 1800 blood biochemistry analyzer/IFCC modified (Siemens) according to standard protocols such as described before.

In order to summarize the results of the experiments, Figure 2 shows the development of ALT activity in the tested rats. As can be seen there, there is an expected increase in ALT activity upon parenteral nutrition over 17 days with LE 10%. In comparison, ALT activity does not increase in study groups where a choline source is present in the lipid emulsion. However, the effect is most notable in the presence of GPC, where a clear drop in ALT activity can be determined. In contrast, choline chloride and CDP-choline rather provide for a stable situation where ALT activity neither significantly increases or decreases. Notably, phosphatidylcholine which is contained in LE lipid emulsion(s) does not seem to have any effect, confirming that choline cannot make use of the choline provided in the form of phosphorylcholine in lipid emulsions.

After having identified GPC as a surprisingly effective choline derivative for the reduction of ALT activity and thus liver damage, another experiment focused on the synergy between GPC and the PUFAs DHA and ARA. Lipid emulsions as described in Example 1 were used as further shown in Table III.

Figure 3 summarizes the results for ALT activity in terms of the change in activity of the normal value. The test includes one experiment with a composition wherein the phytosterol content of the lipid emulsion was reduced as indicated in Fig. 3 according to the Step#3 setup. The results also demonstrate that both the saline solution and lipid emulsion ("LE") without choline source and normal phytosterol content lead to an increase in ALT activity, evidencing a progressive deterioration of the liver. In contrast, both lipid emulsions with GPC (40 mmol/L), irrespective of the phytosterol content, result in a significant decrease of ALT activity. Phytosterol doses are shown in Figure 3. Phytosterol was contained in the LE 13% lipid emulsion in a concentration of from 40 µg/mL to 120 µg/L. Notably, phosphatidylcholine which is contained in the lipid emulsion(s) used here does not seem to have any effect, confirming that the body cannot make use of the choline provided in the form of phosphorylcholine in lipid emulsions.

### Example 3: Determination of histopathological grades

### Example 3.1: Preparation of histological slides

A part of the liver was preserved in buffered formalin, the infusion sites and the sampled macroscopic lesions were trimmed according to the RITA guidelines, where applicable (Ruehl-Fehlert et al., Exp Toxic Pathol 2003; 55, 91-106), embedded in paraffin wax, sectioned at a thickness of approximately four microns and stained with hematoxylin-eosin. The frozen liver sample was cryosectioned in slides of approximately five microns and stained by Oil Red O. Tissue processing was performed at Citoxlab, France.

### Example 3.2: Microscopic examination

A microscopic examination was performed on both slides (hematoxylin eosin and Oil Red O) of the liver, the infusion sites and the macroscopic lesions from all animals of the study. Peer review was performed on at least 30% of the animals from the high-dose group and on an adequate number of slides from identified target organs to confirm that findings recorded by the Study Pathologist were consistent and accurate. After completion of the pathology peer review, all tissue slides were returned to Citoxlab France for archiving. During microscopic examinations, representative photographs were made at the discretion of the pathologist.

### Example 3.3: Mean histology vacuolation

Liver samples taken after 8 and 15 days, respectively, were examined as described above and assigned to five grades of hepatocellular vacuolation. Vacuolation was generally diffuse and affected all regions for Grade 5. Periportal areas were lesser affected and the vacuoles were smaller in size for lower grades. Grades have been assigned according to the following definition:
- GRADE 1 =: Minimal/very few/very small vacuoles, scattered.
- GRADE 2 =: Slight/few/small vacuoles, centrilobular to midzonal.
- GRADE 3 =: Moderate/moderate number/moderate size vacuoles, centrilobular to midzonal.
- GRADE 4 =: Marked/many/large vacuoles, centrilobular to periportal.
- GRADE 5 =: Severe/many/large vacuoles, diffuse, all liver regions affected.

Exemplary photographs for each Grade are shown in Figure 6. The histological slides shown are stained with hematoxylin-eosin (HE).

After 8 days, the administration of saline solution results in four of four animals showing signs of liver damage in terms of vacuolation according to Grade 5 (see Table IV). Parenteral nutrition with LE 13% in the presence of DHA and ARA already generates a positive effect on liver histopathology. Out of five animals, two still show Grade 5 vacuolation. However, three of the five animals' livers can be assigned to Grade 4. In the presence of GPC additional to DHA and ARA, the vacuolation of the liver is even more improved. Only one of five animals shows Grade 5 signs of liver damage, whereas another two rats show Grade 4 symptoms and two Grade 3 symptoms. Finally, if only GPC is added to the lipid emulsion, none of the five rats shows Grade 5 damage of the liver. Three show Grade 4, one shows Grade 3 and one even shows Grade 2 vacuolation.

**Table IV: Microscopic changes of the liver after 8 days**

| **Relevant microscopic changes in the liver on Day 8** | | | | |
|---|---|---|---|---|
| **Group** | **1** | **2** | **3** | **4** |
| **Treatment** | **Saline solution** | **LE 13% + DHA + ARA** | **LE 13% + DHA + ARA + GPC** | **LE 13% + GPC** |
| **No. animals** | 4 | 5 | 5 | 5 |

| **Vacuolation; hepatocellular** | | | | |
|---|---|---|---|---|
| **Grade 2** | - | - | - | 1 |
| **Grade 3** | - | - | 2 | 1 |
| **Grade 4** | - | 3 | 2 | 3 |
| **Grade 5** | 4 | 2 | 1 | - |

After 15 days into parenteral nutrition, the above effect was even more pronounced. Whereas the liver condition of those animals receiving saline solution remained as critical as could be expected, the presence of GPC in the lipid emulsion as well as the presence of all of DHA, ARA and GPC lead to a clear and significant improvement of the liver status of the animal. After 15 days, it becomes evident also that the presence of DHA and ARA within a certain concentration range in addition to GPC even lead to synergistically improved results. Figure 4B summarizes the effects of DHA, ARA and GPC on the development of liver vacuolation, in terms of the above defined grades. The graph shows a significant amelioration of the liver condition in the presence of all DHA, ARA and GPC, wherein GPC seems to have an additional, surprising impact in the already beneficial presence of DHA and ARA in the lipid emulsion.

Identical histopathological tests were performed for comparing the effect of choline derivatives alone, i.e. in the absence of DHA and ARA. Figure 4A summarizes the result of the histopathological examination of the livers of the animal study group. The effects of GPC are significantly better, both in at a high (31 mmol/L) and low (15 mmol/L) GPC concentration. CDP-choline (low concentration, 15 mmol/L) also had an effect. No grades could be assigned to the CDP-choline group with a high concentration (31 mmol/L). The groups which had received choline chloride as part of the lipid emulsion (high and low concentration) fared better than those with only saline or lipid emulsion without any choline derivative. However, the liver damage could not be addressed with said choline chloride supplemented lipid emulsions to any relevant extent.

**Table V: Microscopic changes of the liver after 15 days**

| **Relevant microscopic changes in the liver on Day 15** | | | | |
|---|---|---|---|---|
| **Group** | **1** | **2** | **3** | **4** |
| **Treatment** | **Saline solution** | **LE 13% + DHA + ARA** | **LE 13% + DHA + ARA + GPC** | **LE 13% + GPC** |
| **No. animals** | 5 | 5 | 5 | 5 |

| **Vacuolation; hepatocellular** | | | | |
|---|---|---|---|---|
| **Grade 2** | - | 1 | 1 | 2 |
| **Grade 3** | - | - | 4 | 2 |
| **Grade 4** | - | 2 | - | 1 |
| **Grade 5** | 5 | 2 | - | - |

### Example 4: Development of liver to body weight

The study groups as described in Example 1 were further evaluated with regard to the development of liver weight versus body weight. An increase in liver weight which is not proportional to the increase of body weight is typical for the development of liver damage, specifically steatosis, as described before. Figure 5 shows the results for the liver to body weight ratio in per cent again for those groups of animals that received only saline solution, LE 10%, and the same lipid emulsion with choline derivatives added in concentration as described before ("high": 31 mmol/L). As can be seen, the administration of a lipid emulsion alone leads to an increase in said ratio, indicating a gain in liver weight due to liver damage (steatosis). The addition of choline chloride or CDP-choline both lead to a reduction of the ratio and thus an improvement of the liver status. However, the most pronounced reduction of the ratio is again achieved with GPC. Again, phosphatidylcholine which is contained in the lipid emulsion(s) does not seem to have any effect.

### Example 5: Stability of lipid emulsions in the presence of choline derivatives

Besides investigating into the efficacy of choline derivatives in addressing liver damage, the lipid emulsions prepared as described in Example 1 were further assessed for their respective stability after sterilization (see Table VI). It was found that the stability of the lipid emulsions was improved in the presence of GPC compared to choline chloride or CDP-choline. In fact, choline chloride and CDP-choline were found to destabilize the lipid emulsions during sterilization (heat). Lipid emulsions containing either choline chloride or CDP-choline resulted in a phase separation of the lipid emulsions during sterilization. In the presence of GPC, the lipid emulsions remained stable.

**Table VI: Stability of lipid emulsions upon sterilization in the presence of different choline derivatives**

| **Choline Derivative** | **Concentration (mmol/L)** | **Lipid Emulsions tested: LE (%)** | **Comment** |
|---|---|---|---|
| Choline Chloride | 15 | 10, 13, 20 | Phase separation during sterilization |
| Glycerophosphocholine | 30 | 10, 20 | Stable emulsion |
| CDP-choline | 30 | 10 | Phase separation during sterilization |

The invention further discloses the following preferred embodiments:
1. A multi-chamber container for parenteral administration comprising:
   (i) a carbohydrate formulation present in a first chamber;
   (ii) an amino acid formulation present in a second chamber; and
   (iii) a lipid formulation present in a third chamber;
   wherein:
   the lipid formulation comprises an aqueous phase and about 5% to about 35% by weight of an oil phase based on the total weight of the lipid formulation;
   the lipid formulation is present in the form of an oil-in-water emulsion;
   the oil phase comprises docosahexaenoic acid (DHA) obtained from a single cell source, wherein the single cell source is an extract of microalgae and wherein DHA is present in a concentration of from 0.1 g to 5.0 g per 100 g of oil phase;
   the lipid formulation includes about 70 mg phytosterols or less per 100 g of oil phase; and
   the lipid formulation is essentially free of water soluble forms of choline and/or arachidonic acid (ARA).
2. The multi-chamber container of embodiment 1, wherein DHA is present in a concentration of from 0.25 g to 3.0 g per 100 g of oil phase.
3. The multi-chamber container of embodiment 1 or 2, wherein DHA is present in a concentration of from 1.5 g to 2.5 g per 100 g of oil phase.
4. The multi-chamber container of embodiment 1, 2, or 3, wherein DHA is present in triglyceride form or ethyl ester form, preferably triglyceride form.
5. The multi-chamber container of embodiment 1, 2, 3, or 4, wherein the lipid formulation comprises eicosapentaenoic acid (EPA) and the ratio of DHA to EPA in the lipid formulation is 10:1 to 1000:1 (w/w).
6. The multi-chamber container of embodiment 1, 2, 3, 4, or 5, wherein the lipid formulation comprises EPA and the ratio of DHA to EPA in the lipid formulation is 10:1 to 200:1.
7. The multi-chamber container of embodiment 1, 2, 3, 4, 5, or 6 wherein the lipid formulation comprises EPA and the ratio of DHA to EPA in the lipid formulation is 20:1 to 150:1.
8. The multi-chamber container of embodiment 5, 6, or 7, wherein DHA and/or EPA are present in triglyceride form or ethyl ester form, preferably triglyceride form.
9. The multi-chamber container of embodiment 1, 2, 3, or 4, wherein the lipid formulation is essentially free of EPA.
10. The multi-chamber container of embodiment 1, 2, 3, 4, 5, 6, 7, 8, or 9, wherein the microalgae is *Crypthecodinium cohnii* or *Schizochytrium sp,* preferably *Schizochytrium sp.*
11. The multi-chamber container of embodiment 1, 2, 3, 4, 5, 6, 7, 8, 9, or 10, wherein the lipid formulation includes about 15 mg to about 35 mg phytosterols per 100 g of oil phase, preferably about 25 mg phytosterols or less per 100 g of oil phase.
12. The multi-chamber container of embodiment 1, 2, 3, 4, 5, 6, 7, 8, 9, 10, or 11, wherein the lipid formulation comprises one or more pharmaceutically acceptable surfactants selected from the group consisting of phospholipids, oleate salts, and combinations thereof.
13. The multi-chamber container of embodiment 12, wherein the phospholipid is selected from the group consisting of egg phosphatide and soy lecithin.
14. The multi-chamber container of embodiment 1, 2, 3, 4, 5, 6, 7, 8, 9, 10, 11, 12, or 13, wherein the lipid formulation comprises one or more pharmaceutically acceptable isotonic agents.
15. The multi-chamber container of embodiment 14, wherein the pharmaceutically acceptable isotonic agent is glycerol.
16. The multi-chamber container of embodiment 1, 2, 3, 4, 5, 6, 7, 8, 9, 10, 11, 12, 13, 14, or 15, wherein the lipid formulation comprises one or more pharmaceutically acceptable antioxidants selected from the group consisting of tocopherols, ascorbyl palmitate, and combinations thereof.
17. The multi-chamber container of embodiment 1, 2, 3, 4, 5, 6, 7, 8, 9, 10, 11, 12, 13, 14, 15, or 16, wherein the lipid formulation has a pH in the range of about 6 to about 9, preferably about 7 to about 8.
18. The multi-chamber container of embodiment 1, 2, 3, 4, 5, 6, 7, 8, 9, 10, 11, 12, 13, 14, 15, 16, or 17, wherein the carbohydrate formulation and/or the amino acid formulation comprises at least one water-soluble form of choline selected from the group consisting of choline chloride, choline bitartrate, choline citrate, choline gluconate, choline malate, choline cytidine diphosphate choline (CDP) salt and glycerophosphocholine, preferably glycerophosphocholine.
19. The multi-chamber container of embodiment 18, wherein the at least one water-soluble form of choline is present in a concentration of from 20 mg to 2000 mg choline equivalent per liter of a reconstituted multi-chamber container, such as 30 mg to 1000 mg choline equivalent per liter of a reconstituted multi-chamber container.
20. A method of treating patients who require parenteral nutrition when oral and enteral nutrition is not possible, insufficient or contraindicated with a multi-chamber container of embodiment 1, 2, 3, 4, 5, 6, 7, 8, 9, 10, 11, 12, 13, 14, 15, 16, 17, 18, or 19.
21. The method of embodiment 20, wherein the patients are pediatric patients.
22. A lipid formulation for parenteral administration comprising:
   an aqueous phase and
   about 5% to about 35% by weight of an oil phase based on the total weight of the lipid formulation;
   wherein:
      the lipid formulation is present in the form of an oil-in-water emulsion;
      the aqueous phase comprises at least one pharmaceutically acceptable water soluble form of choline;
      the oil phase comprises docosahexaenoic acid (DHA);
      the lipid formulation includes about 70 mg phytosterols or less per 100 g of oil phase; and
      the lipid formulation is essentially free of arachidonic acid (ARA).
23. The lipid formulation of embodiment 22, wherein the DHA is obtained from a single cell source, wherein the single cell source is an extract of microalgae.
24. The lipid formulation of embodiment 23, wherein the microalgae is *Crypthecodinium cohnii* or *Schizochytrium sp,* preferably *Schizochytrium sp.*
25. The lipid formulation of embodiment 22, 23, or 24, wherein DHA is present in a concentration of from 0.25 g to 3.0 g per 100 g of oil phase.
26. The lipid formulation of embodiment 22, 23, 24, or 25, wherein DHA is present in a concentration of from 1.5 g to 2.5 g per 100 g of oil phase.
27. The lipid formulation of embodiment 22, 23, 24, 25, or 26, wherein DHA is present in triglyceride form or ethyl ester form, preferably triglyceride form.
28. The lipid formulation of embodiment 22, 23, 24, 25, 26, or 27, wherein the lipid formulation comprises eicosapentaenoic acid (EPA) and the ratio of DHA to EPA in the lipid formulation is 10:1 to 1000:1 (w/w).
29. The lipid formulation of embodiment 22, 23, 24, 25, 26, 27, or 28, wherein the lipid formulation comprises EPA and the ratio of DHA to EPA in the lipid formulation is 10:1 to 200:1 (w/w).
30. The lipid formulation of embodiment 22, 23, 24, 25, 26, 27, 28, or 29, wherein the lipid formulation comprises EPA and the ratio of DHA to EPA in the lipid formulation is 20:1 to 150:1 (w/w).
31. The lipid formulation of embodiment 28, 29, or 30, wherein DHA and/or EPA are present in triglyceride form or ethyl ester form, preferably triglyceride form.
32. The lipid formulation of embodiment 22, 23, 24, 25, 26, or 27, wherein the lipid formulation is essentially free of EPA.
33. The lipid formulation of embodiment 22, 23, 24, 25, 26, 27, 28, 29, 30, 31, or 32, wherein the lipid formulation includes 15 mg to 35 mg phytosterols per 100 g of oil phase, preferably about 25 mg phytosterols or less per 100 g oil phase.
34. The lipid formulation of embodiment 22, 23, 24, 25, 26, 27, 28, 29, 30, 31, 32, or 33, wherein the lipid formulation comprises one or more pharmaceutically acceptable surfactants selected from the group consisting of phospholipids, oleate salts, and combinations thereof.
35. The lipid formulation of embodiment 34, wherein the phospholipid is selected from the group consisting of egg phosphatide and soy lecithin.
36. The lipid formulation of embodiment 22, 23, 24, 25, 26, 27, 28, 29, 30, 31, 32, 33, 34, or 35, wherein the lipid formulation comprises one or more pharmaceutically acceptable isotonic agents.
37. The lipid formulation of embodiment 36, wherein the pharmaceutically acceptable isotonic agent is glycerol.
38. The lipid formulation of embodiment 22, 23, 24, 25, 26, 27, 28, 29, 30, 31, 32, 33, 34, 35, 36, or 37, wherein the lipid formulation comprises one or more pharmaceutically acceptable antioxidants selected from the group consisting of tocopherols, ascorbyl palmitate, and combinations thereof.
39. The lipid formulation of embodiment 22, 23, 24, 25, 26, 27, 28, 29, 30, 31, 32, 33, 34, 35, 36, 37, or 38, wherein the lipid formulation has a pH in the range of about 6 to about 9, preferably about 7 to about 8.
40. The lipid formulation of embodiment 22, 23, 24, 25, 26, 27, 28, 29, 30, 31, 32, 33, 34, 35, 36, 37, 38, or 39, wherein the at least one choline is selected from the group consisting of choline chloride, choline bitartrate, choline citrate, choline gluconate, choline malate, choline cytidine diphosphate choline (CDP) salt and glycerophosphocholine, preferably glycerophosphocholine.
41. The lipid formulation of embodiment 22, 23, 24, 25, 26, 27, 28, 29, 30, 31, 32, 33, 34, 35, 36, 37, 38, 39, or 40, wherein the at least one choline is present in a concentration of from 0.1 g to 12 g of choline equivalent per liter of lipid emulsion, for example, from 0.2 g to 10 g of choline equivalent per liter of lipid emulsion, from 3 g to 6 g of choline equivalent per liter of lipid emulsion, from 0.5 to 4 g of choline equivalent per liter of lipid emulsion, from 0.1 g to 0.5 g of choline equivalent per liter of lipid emulsion, from 0.5 g to 1 g of choline equivalent per liter of lipid emulsion, from 1 g to 2 g of choline equivalent per liter of lipid emulsion, from 2 g to 3 g of choline equivalent per liter of lipid emulsion, from 3 g to 4 g of choline equivalent per liter of lipid emulsion, from 4 g to 5 g of choline equivalent per liter of lipid emulsion, from 5 g to 6 g of choline equivalent per liter of lipid emulsion, from 6 g to 7 g of choline equivalent per liter of lipid emulsion, from 7 g to 8 g of choline equivalent per liter of lipid emulsion, from 8 g to 9 g of choline equivalent per liter of lipid emulsion, from 9 g to 10 g of choline equivalent per liter of lipid emulsion, from 10 g to 11 g of choline equivalent per liter of lipid emulsion, and/or from 11 g to 12 g of choline equivalent per liter of lipid emulsion.
42. The lipid formulation of embodiment 22, 23, 24, 25, 26, 27, 28, 29, 30, 31, 32, 33, 34, 35, 36, 37, 38, 39, 40, or 41, wherein the at least one choline is present in a concentration of from 0.2 g to 10 g of choline equivalent per liter of lipid emulsion.
43. The lipid formulation of embodiment 22, 23, 24, 25, 26, 27, 28, 29, 30, 31, 32, 33, 34, 35, 36, 37, 38, 39, 40, 41, or 42, wherein the at least one choline is present in a concentration of from 3 g to 6 g of choline equivalent per liter of lipid emulsion.
44. A method of treating patients who require parenteral nutrition when oral and enteral nutrition is not possible, insufficient or contraindicated with a lipid formulation of embodiment 22, 23, 24, 25, 26, 27, 28, 29, 30, 31, 32, 33, 34, 35, 36, 37, 38, 39, 40, 41, 42, or 43.
45. The method of embodiment 44, wherein the patients are pediatric patients.
46. A multi-chamber container for parenteral administration comprising:
   (i) a carbohydrate formulation present in a first chamber;
   (ii) an amino acid formulation present in a second chamber; and
   (iii) the lipid formulation of embodiment 22, 23, 24, 25, 26, 27, 28, 29, 30, 31, 32, 33, 34, 35, 36, 37, 38, 39, 40, 41, 42, or 43 present in a third chamber.
47. A lipid formulation for parenteral administration comprising:
   an aqueous phase and
   about 5% to about 35% by weight of an oil phase based on the total weight of the lipid formulation;
   wherein:
      the lipid formulation is present in the form of an oil-in-water emulsion;
      the aqueous phase comprises at least one pharmaceutically acceptable water soluble form of choline;
      the oil phase comprises docosahexaenoic acid (DHA) and arachidonic acid (ARA);
      the ratio of DHA to ARA in the lipid formulation is from 10:1 to 1:5 (w/w); and
      the lipid formulation includes about 70 mg phytosterols or less per 100 g of oil phase.
48. The lipid formulation of embodiment 47, wherein the ratio of DHA to ARA in the lipid formulation is 1:1 to 1:3 (w/w).
49. The lipid formulation of embodiment 47 or 48, wherein DHA is obtained from a single cell source, wherein the single source is an extract of microalgae.
50. The lipid formulation of embodiment 49, wherein the microalgae is *Crypthecodinium cohnii* or *Schizochytrium sp,* preferably *Schizochytrium sp.*
51. The lipid formulation of embodiment 47, 48, 49, or 50, wherein ARA is obtained from a single cell source, wherein the cell source is fungi.
52. The lipid formulation of embodiment 51, wherein the fungus is *Mortierella alpina.*
53. The lipid formulation of embodiment 47, 48, 49, 50, 51, or 52, wherein DHA and/or ARA are present in triglyceride form or ethyl ester form, preferably triglyceride form.
54. The lipid formulation of embodiment 47, 48, 49, 50, 51, 52, or 53, wherein ARA is present in a concentration range of from 0.1 g to 15 g per 100 g of oil phase.
55. The lipid formulation of embodiment 47, 48, 49, 50, 51, 52, 53, or 54, wherein ARA is present in a concentration range of from 1.5 g to 7.5 g per 100 g of oil phase.
56. The lipid formulation of embodiment 47, 48, 49, 50, 51, 52, 53, 54, or 55, wherein DHA is present in a concentration of from 0.25 g to 3.0 g per 100 g of oil phase.
57. The lipid formulation of embodiment 47, 48, 49, 50, 51, 52, 53, 54, 55, or 56, wherein DHA is present in a concentration of from 1.5 g to 2.5 g per 100 g of oil phase.
58. The lipid formulation of embodiment 47, 48, 49, 50, 51, 52, 53, 54, 55, 56, or 57, wherein the lipid formulation comprises EPA and wherein the ratio of DHA to EPA in the lipid formulation is 10:1 to 1000:1 (w/w).
59. The lipid formulation of embodiment 47, 48, 49, 50, 51, 52, 53, 54, 55, 56, 57, or 58, wherein the lipid formulation comprises EPA and the ratio of DHA to EPA in the lipid formulation is 10:1 to 200:1 (w/w).
60. The lipid formulation of embodiment 47, 48, 49, 50, 51, 52, 53, 54, 55, 56, 57, 58, or 59, wherein the lipid formulation comprises EPA and the ratio of DHA to EPA in the lipid formulation is 20:1 to 150:1 (w/w).
61. The lipid formulation of embodiment 47, 48, 49, 50, 51, 52, 53, 54, 55, 56, 57, 58, 59, or 60, wherein DHA and/or EPA are present in triglyceride form or ethyl ester form, preferably triglyceride form.
62. The lipid formulation of embodiment 47, 48, 49, 50, 51, 52, 53, 54, 55, 56, or 57, wherein the lipid formulation is essentially free of EPA.
63. The lipid formulation of embodiment 47, 48, 49, 50, 51, 52, 53, 54, 55, 56, 57, 58, 59, 60, 61, or 62, wherein the lipid formulation includes 15 mg to 35 mg phytosterols per 100 g of oil phase, preferably about 25 mg phytosterols or less per 100 g oil phase.
64. The lipid formulation of embodiment 47, 48, 49, 50, 51, 52, 53, 54, 55, 56, 57, 58, 59, 60, 61, 62, or 63, wherein the lipid formulation comprises one or more pharmaceutically acceptable surfactants selected from the group consisting of phospholipids, oleate salts, and combinations thereof.
65. The lipid formulation of embodiment 64, wherein the phospholipid is selected from the group consisting of egg phosphatide and soy lecithin.
66. The lipid formulation of embodiment 47, 48, 49, 50, 51, 52, 53, 54, 55, 56, 57, 58, 59, 60, 61, 62, 63, 64, or 65, wherein the lipid formulation comprises one or more pharmaceutically acceptable isotonic agents.
67. The lipid formulation of embodiment 66, wherein the pharmaceutically acceptable isotonic agent is glycerol.
68. The lipid formulation of embodiment 47, 48, 49, 50, 51, 52, 53, 54, 55, 56, 57, 58, 59, 60, 61, 62, 63, 64, 65, 66, or 67, wherein the lipid formulation comprises one or more pharmaceutically acceptable antioxidants selected from the group consisting of tocopherols, ascorbyl palmitate, and combinations thereof.
69. The lipid formulation of embodiment 47, 48, 49, 50, 51, 52, 53, 54, 55, 56, 57, 58, 59, 60, 61, 62, 63, 64, 65, 66, 67, or 68, wherein the lipid formulation has a pH in the range of about 6 to about 9, preferably of about 7 to about 8.
70. The lipid formulation of embodiment 47, 48, 49, 50, 51, 52, 53, 54, 55, 56, 57, 58, 59, 60, 61, 62, 63, 64, 65, 66, 67,, wherein the at least one choline is selected from the group consisting of choline chloride, choline bitartrate, choline citrate, choline gluconate, choline malate, choline cytidine diphosphate choline (CDP) salt and glycerophosphocholine, preferably glycerophosphocholine.
71. The lipid formulation of embodiment 47, 48, 49, 50, 51, 52, 53, 54, 55, 56, 57, 58, 59, 60, 61, 62, 63, 64, 65, 66, 67, 68, 69, or 70, wherein the at least one choline is present in a concentration of from 0.1 g to 12 g of choline equivalent per liter of lipid emulsion, for example, from 0.2 g to 10 g of choline equivalent per liter of lipid emulsion, from 3 g to 6 g of choline equivalent per liter of lipid emulsion, from 0.5 to 4 g of choline equivalent per liter of lipid emulsion, from 0.1 g to 0.5 g of choline equivalent per liter of lipid emulsion, from 0.5 g to 1 g of choline equivalent per liter of lipid emulsion, from 1 g to 2 g of choline equivalent per liter of lipid emulsion, from 2 g to 3 g of choline equivalent per liter of lipid emulsion, from 3 g to 4 g of choline equivalent per liter of lipid emulsion, from 4 g to 5 g of choline equivalent per liter of lipid emulsion, from 5 g to 6 g of choline equivalent per liter of lipid emulsion, from 6 g to 7 g of choline equivalent per liter of lipid emulsion, from 7 g to 8 g of choline equivalent per liter of lipid emulsion, from 8 g to 9 g of choline equivalent per liter of lipid emulsion, from 9 g to 10 g of choline equivalent per liter of lipid emulsion, from 10 g to 11 g of choline equivalent per liter of lipid emulsion, and/or from 11 g to 12 g of choline equivalent per liter of lipid emulsion.
72. The lipid formulation of embodiment 47, 48, 49, 50, 51, 52, 53, 54, 55, 56, 57, 58, 59, 60, 61, 62, 63, 64, 65, 66, 67, 68, 69, 70, or 71, wherein the at least one choline is present in a concentration of from 0.2 g to 10 g of choline equivalent per liter of lipid emulsion.
73. The lipid formulation of embodiment 47, 48, 49, 50, 51, 52, 53, 54, 55, 56, 57, 58, 59, 60, 61, 62, 63, 64, 65, 66, 67, 68, 69, 70, 71, or 72, wherein the at least one choline is present in a concentration of from 3 g to 6 g of choline equivalent per liter of lipid emulsion.
74. The lipid formulation of embodiment 47, 48, 49, 50, 51, 52, 53, 54, 55, 56, 57, 58, 59, 60, 61, 62, 63, 64, 65, 66, 67, 68, 69, 70, 71, 72, or 73, wherein the choline is present in a concentration of from 0.5 to 4 g of choline equivalent per liter of lipid emulsion.
75. A method of treating pediatric patients who require parenteral nutrition when oral and enteral nutrition is not possible, insufficient or contraindicated with a lipid formulation of embodiment 47, 48, 49, 50, 51, 52, 53, 54, 55, 56, 57, 58, 59, 60, 61, 62, 63, 64, 65, 66, 67, 68, 69, 70, 71, 72, 73, or 74.
76. A multi-chamber container for parenteral administration comprising:
   (i) a carbohydrate formulation present in a first chamber;
   (ii) an amino acid formulation present in a second chamber; and
   (iii) the lipid formulation of embodiment 47, 48, 49, 50, 51, 52, 53, 54, 55, 56, 57, 58, 59, 60, 61, 62, 63, 64, 65, 66, 67, 68, 69, 70, 71, 72, 73, or 74 present in a third chamber.

## Claims

1. A parenteral nutrition product for parenteral administration comprising:
a multi-chamber container having a first chamber, a second chamber and a third chamber; and
(i) a carbohydrate formulation present in the first chamber;
(ii) an amino acid formulation present in the second chamber; and
(iii) a lipid formulation present in the third chamber;
wherein:
the lipid formulation comprises an aqueous phase and 5% to 35% by weight of an oil phase based on the total weight of the lipid formulation;
the lipid formulation is present in the form of an oil-in-water emulsion;
the oil phase comprises docosahexaenoic acid (DHA), wherein DHA is present in a concentration of from 0.1 g to 5.0 g per 100 g of oil phase;
the lipid formulation includes 15 mg to 70 mg phytosterols per 100 g of oil phase; and
the lipid formulation is free of water-soluble forms of choline, wherein the carbohydrate formulation and/or the amino acid formulation comprises at least one water-soluble form of choline.

2. The multi-chamber container of Claim 1, wherein DHA is present in a concentration of from 0.25 g to 3.0 g per 100 g of oil phase.

3. The multi-chamber container of Claim 1 or 2, wherein DHA is present in a concentration of from 1.5 g to 2.5 g per 100 g of oil phase.

4. The multi-chamber container of Claim 1, 2, or 3, wherein DHA is present in triglyceride form or ethyl ester form, preferably triglyceride form.

5. The multi-chamber container of Claim 1, 2, 3, or 4, wherein the lipid formulation comprises eicosapentaenoic acid (EPA) and the ratio of DHA to EPA in the lipid formulation is 10:1 to 1000:1 (w/w).

6. The multi-chamber container of Claim 1, 2, 3, 4, or 5, wherein the lipid formulation comprises EPA and the ratio of DHA to EPA in the lipid formulation is 10:1 to 200:1.

7. The multi-chamber container of Claim 1, 2, 3, 4, 5, or 6 wherein the lipid formulation comprises EPA and the ratio of DHA to EPA in the lipid formulation is 20:1 to 150:1.

8. The multi-chamber container of Claim 5, 6, or 7, wherein DHA and/or EPA are present in triglyceride form or ethyl ester form, preferably triglyceride form.

9. The multi-chamber container of Claim 1, 2, 3, or 4, wherein the lipid formulation is essentially free of EPA.

10. The multi-chamber container of Claim 1, 2, 3, 4, 5, 6, 7, 8, or 9, wherein the microalgae is *Crypthecodinium cohnii* or *Schizochytrium sp,* preferably *Schizochytrium sp.*

11. The multi-chamber container of Claim 1, 2, 3, 4, 5, 6, 7, 8, 9, or 10, wherein the lipid formulation includes about 15 mg to about 35 mg phytosterols per 100 g of oil phase, preferably about 25 mg phytosterols or less per 100 g of oil phase.

12. The multi-chamber container of Claim 1, 2, 3, 4, 5, 6, 7, 8, 9, 10, or 11, wherein the lipid formulation comprises one or more pharmaceutically acceptable surfactants selected from the group consisting of phospholipids, oleate salts, and combinations thereof.

13. The multi-chamber container of Claim 12, wherein the phospholipid is selected from the group consisting of egg phosphatide and soy lecithin.

14. The multi-chamber container of Claim 1, 2, 3, 4, 5, 6, 7, 8, 9, 10, 11, 12, or 13, wherein the lipid formulation comprises one or more pharmaceutically acceptable isotonic agents.

15. The multi-chamber container of Claim 14, wherein the pharmaceutically acceptable isotonic agent is glycerol.
